# EUROPÄISCHE PATENTSCHRIFT

(11) **EP 2 582 307 B1**
(45) Veröffentlichungstag und Bekanntmachung des Hinweises auf die Patenterteilung: **08.04.2015**
(21) Anmeldenummer: 11724240.4
(22) Anmeldetag: 10.06.2011
(51) Int. Cl.: A61B 17/00, A61B 17/16

(54) **Gasbetriebenes chirurgisches Instrument**
Gas-powered surgical instrument
Instrument chirurgical actionné par un gaz comprimé

(30) Priorität: 16.06.2010 DE 102010017395
(43) Veröffentlichungstag der Anmeldung: 24.04.2013
(73) Patentinhaber: Aesculap AG, 78532 Tuttlingen (DE)
(72) Erfinder: SCHOLTEN, Thomas, 78532 Tuttlingen (DE); MAYENBERGER, Rupert, 78239 Rielasingen (DE)
(74) Vertreter: Hoeger, Stellrecht & Partner Patentanwälte
(86) Internationale Anmeldenummer: PCT/EP2011/059647
(87) Internationale Veröffentlichungsnummer: WO 2011/157636

(56) Entgegenhaltungen:
- US-A- 4 349 028

## Beschreibung

Die vorliegende Erfindung betrifft ein druckgasbetriebenes chirurgisches Instrument mit einem Druckgasanschluss zum Verbinden mit einer Druckgasquelle, einem mit einem Druckgas beaufschlagbaren Arbeitskolben, einem Betätigungselement zum Betätigen des Instruments und einer mit dem Betätigungselement und dem Arbeitskolben gekoppelten Regelungseinrichtung zum Regeln einer Vorschubkraft des Arbeitskolbens.

Instrumente der eingangs beschriebenen Art sind beispielsweise aus der US 2007/0213769 A1 sowie der US 4,349,028 bekannt. Sie sind zum Beispiel in Form pneumatischer Knochenstanzen ausgebildet oder als Instrumente zum Verriegeln von Implantaten. Um die Instrumente unabhängig von einer Druckgasquelle nutzen zu können, mit der das Instrument über einen Schlauch verbunden wird, wurde in der DE 203 18 275 U1 ein Instrument vorgeschlagen, bei dem als Druckgasquelle eine mit einem Druckgas gefüllte Kartusche nutzbar ist. Nachteilig bei der Verwendung einer Gaskartusche ist jedoch, dass diese nur ein begrenztes Gasvolumen zur Verfügung stellen kann, wodurch ein Einsatz des Instruments zeitlich oder die Zahl von Betätigungszyklen begrenzt ist.

Es ist daher eine Aufgabe der vorliegenden Erfindung, ein druckgasbetriebenes chirurgisches Instrument der eingangs beschriebenen Art so zu verbessern, dass es mit einem möglichst geringen Druckgasvolumen eine möglichst große Anzahl an Betätigungszyklen oder eine möglichst lange Betätigungszeit ermöglicht.

Diese Aufgabe wird bei einem druckgasbetriebenen chirurgischen Instrument der eingangs beschriebenen Art erfindungsgemäß dadurch gelöst, dass die Regelungseinrichtung derart ausgebildet ist, dass sie einen ersten Arbeitsbereich, in welchem der Arbeitskolben allein aufgrund einer mit dem Betätigungselement aufbringbaren Vorschubkraft in distaler Richtung bewegbar ist, für Vorschubkräfte unterhalb einer vorgegebenen Grenzkraft definiert, und dass die Regelungseinrichtung einen zweiten Arbeitsbereich oberhalb der Grenzkraft definiert, in welchem der Arbeitskolben durch Beaufschlagen mit Druckgas in distaler Richtung bewegbar ist.

Die vorgeschlagene Weiterbildung eines druckgasbetriebenen chirurgischen Instruments ermöglicht es, mit möglichst wenig Druckgas als Fremdenergiequelle auszukommen. Druckgas wird bei der erfindungsgemäß ausgebildeten Regelungseinrichtung nur im zweiten oder pneumatischen Arbeitsbereich genutzt, um den Arbeitskolben zu bewegen, wenn eine bestimmte Grenzkraft überschritten wird. Unterhalb der Grenzkraft wird der Arbeitskolben allein aufgrund einer manuell eingeleiteten Vorschubkraft betätigt. Die vorgeschlagene Ausgestaltung des Instruments hat zudem den Vorteil, dass der Anwender, trotz Unterstützung bei der Betätigung des Instruments mit dem Druckgas, mit dem Instrument taktil arbeiten kann. Das Druckgas wird nur dann genutzt, wenn sehr hohe Betätigungskräfte mit dem Instrument ausgeübt werden müssen, was beispielsweise bei Knochenstanzen oder Instrumenten zum Verriegeln von Implantaten der Fall ist. Für Betätigungswege des Arbeitskolbens, die mit einer nur geringen Kraft möglich sind, und zwar einer Kraft unterhalb der Grenzkraft, wird im ersten oder mechanischen Arbeitsbereich auf die Unterstützung des Druckgases bewusst verzichtet. Insbesondere beim Einsatz von Druckgaskartuschen kann so die Zahl der insgesamt möglichen Betätigungszyklen, beispielsweise im Vergleich zu einem aus der DE 203 18 275 U1 bekannten Instrument, deutlich erhöht werden.

Um die Taktilität des Instruments für einen Anwender zu verbessern, ist es günstig, wenn die Regelungseinrichtung derart ausgebildet ist, dass im zweiten Arbeitsbereich eine durch Beaufschlagen mit Druckgas auf den Arbeitskolben wirkende Vorschubkraft proportional zu einer mit dem Betätigungselement aufbringbaren Vorschubkraft ist. Auch mit zunehmend erforderlicher Betätigungskraft, die durch den Arbeitskolben ausgeübt werden soll, nimmt auch die vom Anwender spürbare Kraft zu. Durch die Proportionalität der durch Beaufschlagen mit Druckgas auf den Arbeitskolben wirkenden Vorschubkraft und der direkt manuell mit dem Betätigungselement aufbringbaren Vorschubkraft bleibt jedoch eine Taktilität für den Anwender auch im zweiten Arbeitsbereich oberhalb der definierten Grenzkraft erhalten, und zwar stufenlos. Optional kann es auch vorteilhaft sein, wenn die Regelungseinrichtung derart ausgebildet ist, dass im zweiten Arbeitsbereich eine durch Beaufschlagen mit Druckgas auf den Arbeitskolben in Kombination oder im Zusammenwirken mit einem Stützelement wirkende Vorschubkraft proportional zu einer mit dem Betätigungselement aufbringbaren Vorschubkraft ist. Insbesondere kann das Stützelement im ersten Arbeitsbereich vom Arbeitskolben entkoppelt und relativ zu diesem beweglich, im zweiten Arbeitsbereich mit dem Arbeitskolben axial gekoppelt sein.

Damit auf einfache Weise der Arbeitskolben mit einem Druckgas beaufschlagt werden kann, ist es günstig, wenn die Regelungseinrichtung einen mit Druckgas beaufschlagbaren Arbeitsraum umfasst, welcher in Richtung auf den Arbeitskolben hin mindestens teilweise offen ist. So kann das in den Arbeitsraum eingeleitete Druckgas mindestens auf einen Teil des Arbeitskolbens direkt einwirken und bei Überschreiten eines der Grenzkraft zugeordneten Grenzdrucks den Arbeitskolben bewegen.

Vorteilhafterweise ist der Arbeitskolben in einem Arbeitszylinder verschiebbar geführt. Auf diese Weise kann eine definierte Bewegung des Arbeitskolbens relativ zum Instrument sichergestellt werden.

Um Druckgasverluste zu minimieren, ist es vorteilhaft, wenn der Arbeitskolben und der Arbeitszylinder relativ zueinander abgedichtet sind.

Vorzugsweise umfasst das Instrument ein Stützelement, welches mit dem Arbeitskolben im ersten und/oder zweiten Arbeitsbereich zusammenwirkt. Insbesondere kann sich der Arbeitskolben am Stützelement in mindestens einem der Arbeitsbereiche abstützen und/oder mit diesem koppelbar sein. Dadurch lassen sich insbesondere ein rein mechanisch betätigbarer Arbeitsbereich von einem ausschließlich durch Druckgas betriebenen Arbeitsbereich entkoppeln. Alternativ oder zusätzlich kann durch eine Kopplung des Stützelements und des Arbeitskolbens insbesondere eine Wirkfläche für das Druckgas ändern, zum Beispiel im gekoppelten Zustand vergrößern. Dadurch kann beispielsweise eine Vorschubkrafterhöhung zugeschaltet werden beim Übergang von einem ersten in einen zweiten Arbeitsbereich des Instruments.

Damit der Arbeitskolben auf einfache Weise direkt durch Einleiten einer manuellen Betätigungskraft verschoben werden kann, ist es günstig, wenn sich der Arbeitskolben im ersten Arbeitsbereich am Stützelement abstützt. Beispielsweise kann bei einer Bewegung des Stützelements in distaler Richtung der sich daran abstützende Arbeitskolben ebenfalls in distaler Richtung bewegt werden.

Vorzugsweise ist der Arbeitskolben im zweiten Arbeitsbereich mit dem Stützelement gekoppelt. Beispielsweise kann durch eine Ankopplung des Stützelements an den Arbeitskolben eine Wirkfläche für das Druckgas erhöht werden, so dass im gekoppelten Zustand zum Beispiel eine Vorschubkraft durch entsprechende Druckgasbeaufschlagung erhöht, quasi zugeschaltet, werden kann. Insbesondere kann der Arbeitskolben im ersten Arbeitsbereich vom Stützelement entkoppelt sein.

Eine besonders kompakte Ausbildung des Instruments kann dadurch erreicht werden, dass der Arbeitsraum am Stützelement ausgebildet ist. Insbesondere bei einem bewegbar angeordneten Stützelement kann so der Arbeitsraum "mitwandern", so dass nur das am Stützelement definierte Volumen des Arbeitsraums in einem ersten Schritt mit Druckgas beaufschlagt werden muss, um den Arbeitskolben vom Stützelement weg in distaler Richtung zu bewegen.

Manuelle Betätigungskräfte, die über das Betätigungselement aufgebracht werden, können so direkt vom Stützelement auf den Arbeitskolben übertragen werden, wenn sich der Arbeitskolben im ersten Arbeitsbereich direkt an dem Stützelement abstützt.

Auf eine Druckgasbetätigung des Arbeitskolbens kann insbesondere dann verzichtet werden, wenn das Stützelement im ersten Arbeitsbereich nur aufgrund der mit dem Betätigungselement aufbringbaren Vorschubkraft in distaler Richtung bewegbar ist.

Für ein optimales Zusammenwirken zwischen dem Stützelement und dem Arbeitskolben ist es günstig, wenn das Stützelement mindestens abschnittsweise im Arbeitszylinder geführt ist und wenn das Stützelement und der Arbeitszylinder relativ zueinander abgedichtet sind. Das Stützelement kann so in definierter Weise zumindest teilweise im Arbeitszylinder und durch diesen geführt bewegt werden, ohne dass Druckgas entweichen kann. Optional oder alternativ kann es auch vorteilhaft sein, wenn der Arbeitskolben und das Stützelement relativ zueinander oder aneinander verschiebbar geführt und gegeneinander abgedichtet sind, beispielsweise mit einem am Arbeitskolben und/oder am Stützelement angeordneten, ausgebildeten oder gehaltenen Dichtelement, vorzugsweise in Form eines Dichtringes.

Um eine Bewegung des Stützelements weiter zu unterstützen und definierter Weise zu ermöglichen, ist es günstig, wenn das Instrument ferner eine Führungseinrichtung zum Führen eine Bewegung des Stützelements in distaler und proximaler Richtung umfasst.

Besonders einfach wird der Aufbau des Instruments, wenn die Führungseinrichtung einen Führungszylinder umfasst, in welchem das Stützelement verschiebbar geführt ist. Das Stützelement kann insbesondere kolbenartig ausgebildet sein im Bereich, in dem es im Führungszylinder verschiebbar geführt ist.

Damit möglichst wenig Druckgas aus dem Instrument entweichen kann, ist es vorteilhaft, wenn das Stützelement und die Führungseinrichtung relativ zueinander abgedichtet sind.

Gemäß einer weiteren bevorzugten Ausführungsform der Erfindung kann vorgesehen sein, dass die Regelungseinrichtung eine Feststelleinrichtung zum temporären Festlegen des Stützelements im zweiten Arbeitsbereich umfasst. Mit der Feststelleinrichtung ist es beispielsweise möglich, das Stützelement relativ zum Arbeitszylinder und/oder zum Führungszylinder temporär festzulegen, so dass dann der Arbeitskolben nur durch Druckgasbeaufschlagung in distaler Richtung bewegt werden kann. Das Stützelement insbesondere nur im zweiten Arbeitsbereich am Instrument festzulegen, gestattet es, nur dann, wenn tatsächlich hohe Arbeitskräfte benötigt werden, die entsprechende Druckgasmenge abzurufen und zum Vorschieben des Arbeitskolbens zu nutzen.

Das Stützelement kann auf besonders einfache Weise temporär am Instrument festgelegt werden, wenn die Feststelleinrichtung mindestens ein Feststellglied umfasst zum temporären Festlegen des Stützelements an der Führungseinrichtung oder dem Arbeitskolben. Optional wäre es auch denkbar, die Feststelleinrichtung so auszubilden, dass das Stützelement temporär am Arbeitszylinder festlegbar ist.

Die Konstruktion und die Herstellung des Instruments können weiter vereinfacht werden, wenn das mindestens eine Feststellglied in Form eines Rast- und/oder Klemmelements ausgebildet ist. Das Klemmelement kann insbesondere dann wirken, wenn die vordefinierte Grenzkraft überschritten wird oder ist. Ein Feststellmechanismus lässt sich zudem auf einfache Weise durch eine Rastverbindung mit zueinander korrespondierenden und zusammenwirkenden Rastgliedern ausbilden.

Um das Stützelement in definierter und einfacher Weise am Instrument temporär festzulegen, ist es vorteilhaft, wenn sich das Klemmelement einerseits am Stützelement und andererseits an der Führungseinrichtung oder am Arbeitskolben abstützt. So kann eine direkte Verklemmung des Stützelements mittels des Klemmelements an der Führungseinrichtung oder am Arbeitskolben erreicht werden, und zwar vorzugsweise im zweiten Arbeitsbereich.

Eine besonders einfache Betätigung des Feststellglieds kann dadurch erreicht werden, dass die Feststelleinrichtung ein mit Druckgas beaufschlagbares Kolbenelement umfasst, welches durch Beaufschlagen mit Druckgas in distaler Richtung bewegbar und gegen das mindestens eine Feststellglied drückbar ist zum Verklemmen desselben relativ zum Stützelement und relativ zur Führungseinrichtung oder relativ zum Arbeitskolben. Des Weiteren ermöglicht es diese besondere Ausgestaltung, das zum Bewegen des Arbeitskolbens nutzbare Druckgas gleichzeitig zum Feststellen des Stützelements am Instrument, insbesondere an der Führungseinrichtung oder am Arbeitskolben, zu nutzen, und zwar in Abhängigkeit des im Instrument herrschenden Gasdrucks. Insbesondere kann eine Verklemmung des mindestens einen Feststellglieds relativ zum Stützelement und relativ zur Führungseinrichtung oder relativ zum Arbeitskolben selbsthemmend erfolgen, wobei unter Ausnutzung des Rückstoßprinzips eine Verklemmung der zusammenwirkenden Teile umso stärker ist, je stärker der herrschende Gegendruck ist.

Eine definierte Bewegung des Kolbenelements am Instrument kann insbesondere dadurch erreicht werden, dass das Kolbenelement am Stützelement und/oder an der Führungseinrichtung oder am Arbeitskolben geführt gehalten ist. So kann eine definierte Bewegung des Kolbenelements relativ zum Stützelement und relativ zur Führungseinrichtung oder relativ zum Arbeitskolben sichergestellt werden.

Zur Vermeidung weiterer Druckgasverluste kann es ferner vorteilhaft sein, wenn das Kolbenelement und die Führungseinrichtung und/oder der Arbeitskolben relativ zueinander abgedichtet sind. Vorteilhaft ist es ferner, wenn das Kolbenelement und das Stützelement relativ zueinander abgedichtet sind, um auch hier Druckgasverluste zu vermeiden.

Günstigerweise ist die über das Betätigungselement eingeleitete Betätigungskraft auf ein parallel zum Arbeitskolben bewegbar gelagertes Betätigungsglied übertragbar. Dies ermöglicht es, das Betätigungsglied aufgrund der eingeleiteten Betätigungskraft auch in distaler Richtung, das heißt in Richtung auf den Arbeitskolben hin, zu bewegen, um diesen in distaler Richtung zu bewegen.

Gemäß einer weiteren bevorzugten Ausführungsform der Erfindung kann vorgesehen sein, dass die Regelungseinrichtung einen Druckregler umfasst zum Regeln eines auf den Arbeitskolben wirkenden Gasdrucks in Abhängigkeit einer über das Betätigungselement einleitbaren Betätigungskraft. Der Druckregler ermöglicht es insbesondere, die Grenzkraft festzulegen, die den ersten und den zweiten Arbeitsbereich voneinander trennt beziehungsweise definiert. Insbesondere kann der Druckregler so ausgebildet sein, dass der im Instrument im Druckgas herrschende Druck den Arbeitskolben erst oberhalb der vordefinierten Grenzkraft bewegen kann.

Vorzugsweise ist der Druckregler ausgebildet zum Regeln des auf den Arbeitskolben wirkenden Gasdrucks in Abhängigkeit der auf das Betätigungsglied wirkenden Betätigungskraft. Unabhängig von einer Ausgestaltung des Betätigungselements wird der Gasdruck somit nur in Abhängigkeit der auf das Betätigungsglied tatsächlich wirkenden Betätigungskraft geregelt.

Der Aufbau des Instruments kann weiter vereinfacht werden, wenn der Druckregler ein Ventil umfasst, welches im ersten Arbeitsbereich geschlossen ist. So verhindert der Druckregler, dass der Arbeitskolben im ersten Arbeitsbereich mit Druckgas mit ausreichendem Arbeitsdruck zum Bewegen des Arbeitskolbens beaufschlagt werden kann.

Vorzugsweise umfasst der Druckregler ein erstes Druckglied zum Definieren einer zum Öffnen des Ventils erforderlichen Öffnungskraft. Durch die Art und die Ausgestaltung des Druckglieds kann so die Grenzkraft direkt voreingestellt werden, die überschritten werden muss, um das Ventil zu öffnen.

Eine besonders kompakte Anordnung und Ausbildung der Regelungseinrichtung kann insbesondere dadurch erreicht werden, dass sich das erste Druckglied einerseits am Druckregler und andererseits am Stützelement oder am Arbeitskolben abstützt. Auf diese Weise kann das erste Druckglied direkt auf den Druckregler, optional auch auf das Ventil einwirken.

Um den Druckregler in Abhängigkeit einer Position des Stützelements am Instrument positionieren zu können, ist es günstig, wenn der Druckregler ein Druckreglerkolbenelement umfasst, welches in der Führungseinrichtung verschiebbar geführt ist.

Zur Vermeidung von Druckgasverlusten ist es vorteilhaft, wenn das Druckreglerkolbenelement und die Führungseinrichtung relativ zueinander abgedichtet sind.

Günstigerweise umfasst die Regelungseinrichtung ein zweites Druckglied zum Übertragen einer Betätigungskraft vom Betätigungsglied auf den Druckregler. Das zweite Druckglied kann insbesondere in Abhängigkeit einer auf es einwirkenden Betätigungskraft verformbar sein und zur Übertragung der eingeleiteten Betätigungskraft vom Betätigungsglied auf den Druckregler genutzt werden.

Ferner kann es günstig sein, wenn die Regelungseinrichtung ein drittes Druckglied umfasst, zum Übertragen einer Betätigungskraft vom Betätigungsglied auf den Druckregler. So können zwei unterschiedliche Kraftbereiche durch das zweite und das dritte Druckglied unabhängig voneinander definiert werden. Bei dem dritten Druckglied kann es sich insbesondere um ein weiteres Druckglied handeln. Das Vorhandensein des oben definierten zweiten Druckglieds ist nicht zwingend, sondern optional. Ferner kann das dritte Druckglied auch derart angeordnet und ausgebildet sein, dass mit ihm eine Betätigungskraft direkt auf ein vom Druckregler umfasstes Belüftungsventil übertragbar ist.

Um Druckgas aus dem Arbeitsraum definiert entfernen zu können, ist es vorteilhaft, wenn die Regelungseinrichtung ein Belüftungsventil umfasst zum Belüften des Arbeitsraumes.

Damit das Instrument noch kompakter ausgebildet werden kann, ist es vorteilhaft, wenn der Druckregler das Belüftungsventil umfasst.

Den Druck, ab dem das Belüftungsventil öffnet, kann man auf einfach Weise dadurch einstellen, dass das Belüftungsventil entgegen der Wirkung des dritten Druckglieds öffenbar ist. Mit dem dritten Druckglied kann somit direkt eine Öffnungskraft zum Öffnen des Belüftungsventils definiert werden.

Um die Baugröße des Instruments möglichst kompakt zu halten, umfasst das Druckreglerkolbenelement vorzugsweise das Belüftungsventil oder einen Teil desselben.

Der Arbeitsraum kann in definierter Weise mit Druckgas geflutet werden, wenn das erste, das zweite und das dritte Druckglied derart angeordnet sind, dass eine vom ersten Druckglied ausgeübte Kraft den vom zweiten und dritten Druckglied ausgeübten Kräften entgegenwirkt. Die Druckglieder derart anzuordnen ermöglicht es, eine Betätigungskraft über das zweite und dritte Druckglied einzuleiten, wobei dann, wenn die durch das zweite und dritte Druckglied ausgeübte Kraft etwas größer ist als die vom ersten Druckglied entgegengesetzt wirkende Kraft, das Ventil öffenbar ist zum Einströmen lassen vom Druckgas in den Arbeitsraum.

Um im ersten Arbeitsbereich den Arbeitskolben ohne Beaufschlagen mit Druckgas bewegen zu können, ist es günstig, wenn das erste, das zweite und das dritte Druckglied derart ausgebildet sind, dass eine vom ersten Druckglied ausgeübte Kraft im ersten Arbeitsbereich stets größer ist als die Summe der vom zweiten und dritten Druckglied ausgeübten Kräfte. Es wird so insbesondere verhindert, dass das Ventil zum Herstellen einer Fluidverbindung zwischen dem Arbeitsraum und der Druckgasquelle geöffnet werden kann.

Gemäß einer weiteren bevorzugten Ausführungsform der Erfindung kann eine Rückstelleinrichtung vorgesehen sein zum Überführen des Instruments von einer Arbeitsstellung, in welcher der Arbeitskolben in distaler Richtung bewegt ist, in eine Grundstellung zurück, in welcher der Arbeitskolben seine proximalste Stellung einnimmt. Legt eine Bedienperson das Instrument aus der Hand, ist es so möglich, dass Instrument automatisch wieder in seine Grundstellung zu bringen. Insbesondere kann die Rückstelleinrichtung in Form einer pneumatischen Rückstelleinrichtung ausgebildet sein, gemäß welcher vorgesehen sein kann, dass der Arbeitskolben durch entsprechende Beaufschlagung mit Über- oder Unterdruck in die Grundstellung zurück bewegt wird.

Der Aufbau der Rückstelleinrichtung wird besonders einfach, wenn sie mindestens ein Rückstellglied umfasst, welches sich einerseits an der Führungseinrichtung und andererseits am Arbeitskolben und/oder am Stützelement abstützt zum Ausüben einer in proximaler Richtung wirkenden Kraft auf den Arbeitskolben und/oder das Stützelement. Insbesondere kann es sich distalseitig am Arbeitszylinder und proximalseitig am Arbeitskolben abstützen, so dass durch das Rückstellglied eine in proximaler Richtung wirkende Rückstellkraft auf den Arbeitskolben ausübbar ist, wenn dieser von seiner proximalsten Stellung in distaler Richtung bewegt wurde. Optional kann es sich, insbesondere auch alternativ, distalseitig am Arbeitszylinder und proximalseitig am Stützelement abstützen, so dass durch das Rückstellglied eine in proximaler Richtung wirkende Rückstellkraft auf das Stützelement ausübbar ist, wenn dieses von seiner proximalsten Stellung in distaler Richtung bewegt wurde.

Vorzugsweise umfasst das Instrument ein Gehäuse, wobei an oder in dem Gehäuse der Druckgasanschluss und/oder der Arbeitskolben und/oder das Betätigungselement und/oder die Regelungseinrichtung angeordnet oder ausgebildet sind. Insbesondere lassen sich die genannten Bestandteile des Instruments geschützt oder zumindest teilweise geschützt im Gehäuse anordnen.

Zur Verbesserung der Handhabbarkeit des Instruments ist es vorteilhaft, wenn das Gehäuse einen Griffbereich umfasst und wenn das Betätigungselement am Griffbereich angeordnet oder ausgebildet oder gelagert ist. Eine Bedienperson kann so das Instrument am Griffbereich erfassen und gleichzeitig mit derselben Hand das Betätigungselement bedienen.

Vorzugsweise weist der Griffbereich eine Druckgasbehälteraufnahme für einen Druckgasbehälter auf und ist der Druckgasanschluss im Bereich der oder in der oder an der Druckgasbehälteraufnahme angeordnet oder ausgebildet. Dies gestattet es, den Druckgasbehälter kompakt und ergonomisch optimiert am Instrument anzuordnen. Insbesondere kann die Druckgasbehälteraufnahme verschließbar sein, so dass der Druckgasbehälter vollständig geschützt am Instrument anordenbar ist.

Das chirurgische Instrument, wie es oben beschrieben wurde, kann insbesondere auch in Form eines Instrumentengriffs ausgebildet sein. Um mit dem Instrument chirurgische Eingriffe vornehmen zu können, ist es vorteilhaft, wenn an einem distalen Ende des Arbeitskolbens ein chirurgisches Werkzeugelement angeordnet ist.

Um beispielsweise Gewebe oder Knochen mit dem Instrument bearbeiten zu können, ist es günstig, wenn das chirurgische Werkzeugelement in Form einer Schneide ausgebildet ist.

Damit das Instrument wiederverwendet werden kann, auch wenn das chirurgische Werkzeugelement beschädigt wurde und nicht mehr repariert werden kann, ist es günstig, wenn das Werkzeugelement mit dem Arbeitskolben lösbar verbindbar oder temporär koppelbar ist. Es kann dann je nach Einsatzzweck oder bei Bedarf nach Abnutzung des Werkzeugelements das optimal geeignete Werkzeugelement mit dem Arbeitskolben gekoppelt werden.

Grundsätzlich wäre es denkbar, das chirurgische Instrument über eine Druckgasverbindung mit einer Druckgasquelle, beispielsweise einem Druckgasversorgungssystem in einem Krankenhaus zu verbinden. Um das Instrument unabhängig und insbesondere schlauchverbindungslos nutzen zu können, also ohne störende Verbindungsschläuche die das Instrument mit der Druckgasquelle verbinden, ist es günstig, wenn die Druckgasquelle ein mit einem Druckgas gefüllter Gasbehälter ist und wenn der Gasbehälter mit dem Druckgasanschluss lösbar verbindbar ist. Dies gestattet es, bei Bedarf den leeren Gasbehälter vom Instrument zu entfernen und durch einen vollen Gasbehälter zu ersetzen.

Vorzugweise umfasst das Instrument eine Druckgasquelle in Form eines Druckgasbehälters. Es ist dann, vorausgesetzt der Druckgasbehälter ist mit dem Druckgas gefüllt, ein sofort einsatzbereites Instrument bereitstellbar, welches im ersten Arbeitsbereich ohne Druckgasunterstützung und im zweiten Arbeitsbereich mit Druckgasunterstützung den Arbeitskolben in distaler Richtung bewegen kann.

Vorzugweise ist das Instrument in Form einer Knochenstanze ausgebildet. Knochenstanzen benötigen häufig sehr hohe Kräfte, beispielsweise in der Größenordnung von 750 Newton, um Knochen oder Gewebe zu bearbeiten, zum Beispiel zu durchtrennen. Knochenstanzen weisen zwei zusammenwirkende Werkzeugglieder auf, nämlich ein Ambossteil und eine Schneide, die relativ zum Ambossteil bewegt werden kann. Typischerweise ist das Ambossteil mit dem Gehäuse des Instruments verbunden, die Schneide vorzugsweise mit dem Arbeitskolben des Instruments lösbar verbindbar, um sie bei Bedarf auswechseln zu können. Insbesondere bei Knochenstanzen ist ein maximaler Verschiebeweg durch den Aufbau des Instruments vorgegeben. Er entspricht einem Abstand der Schneide vom Ambossteil in der Grundstellung. Befindet sich zwischen der Schneide und dem Ambossteil kein Gewebe, so wird keine große Kraft benötigt, um die Schneide in Richtung auf und gegen den Ambossteil zu bewegen. In diesem Fall ist eine Druckgasbeaufschlagung nicht erforderlich. Befindet sich jedoch Gewebe oder Knochen zwischen der Schneide und dem Ambossteil, dann wird eine hohe Arbeitskraft benötigt, wenn die Schneide und das Ambossteil den Knochen oder das Gewebe zwischen sich klemmen und durchtrennen sollen. Erst dann wird durch die besondere Ausgestaltung des Instruments Druckgas genutzt, um die zum Bearbeiten des Knochens oder des Gewebes erforderliche Arbeitskraft aufzubringen. So kann durch die Regelungseinrichtung ein Druckgasverbrauch minimiert werden, denn über den Verschiebeweg des Werkzeugselements, in dem dies keine Kraft ausüben muss, kann auf eine Druckgasbeaufschlagung des Arbeitskolbens verzichtet werden.

Gemäß einer alternativen bevorzugten Ausführungsform der Erfindung kann das Instrument in Form eines Implantatverriegelungsinstruments ausgebildet sein. Ähnlich wie bei einer Knochenstanze ist auch bei einem Implantatverriegelungsinstrument nur auf einem relativ kurzen Weg oder Hubbereich des Arbeitskolbens tatsächlich eine hohe Arbeitskraft erforderlich, die ausgeübt werden muss, um zum Beispiel Implantatteile relativ zueinander zu bewegen, insbesondere zu spreizen. Erst im zweiten Arbeitsbereich wird dann tatsächlich Druckgas genutzt, um den Arbeitskolben mit der erforderlichen Kraft zu beaufschlagen, die über eine manuelle Kraftanleitung des Betätigungselements nur schwer oder gar nicht möglich wäre.

Die nachfolgende Beschreibung bevorzugter Ausführungsformen der Erfindung dient im Zusammenhang mit der Zeichnung der näheren Erläuterung. Es zeigen:
- Figur 1:: eine schematische Gesamtansicht eines druckgasbetriebenen chirurgischen Instruments in Form einer Knochenstanze;
- Figur 2:: eine Schnittansicht Längslinie 2 - 2 in Figur 1;
- Figur 3:: eine vergrößerte Ansicht des die Druckregelungseinrichtung umfassenden Teils des Instruments in der Grund- oder Ausgangsstellung;
- Figur 3a:: eine vergrößerte Ansicht des Bereichs A aus Figur 3;
- Figur 3b:: eine vergrößerte Ansicht des Bereichs B aus Figur 3;
- Figur 3c:: eine vergrößerte Ansicht des Bereichs C aus Figur 3;
- Figur 4:: eine Ansicht analog Figur 3 im ersten Arbeitsbereich des Instruments;
- Figur 4a:: eine vergrößerte Ansicht des Bereichs D in Figur 4;

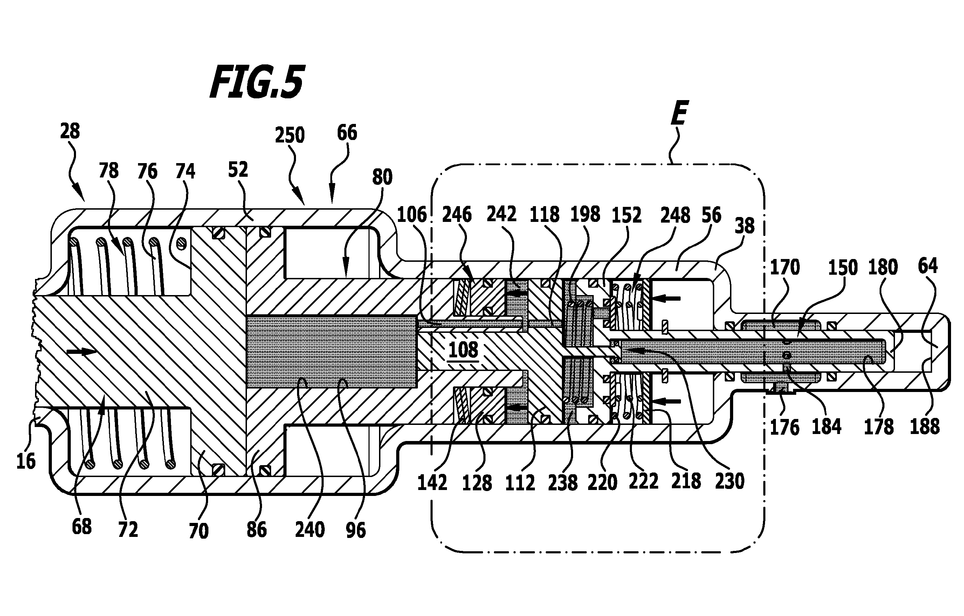

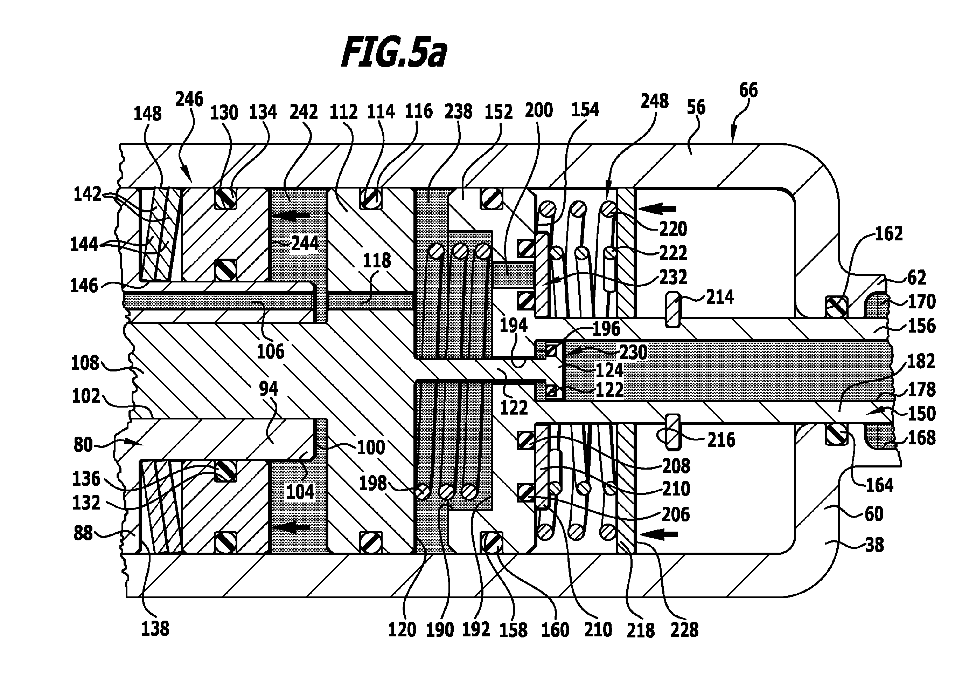

- Figur 5:: eine Ansicht analog Figur 3 noch im ersten Arbeitsbereich des Instruments bei zunehmendem Druckgasdruck im Arbeitsraum;
- Figur 5a:: eine vergrößerte Ansicht des Bereichs E in Figur 5;
- Figur 6:: eine Ansicht analog Figur 3 bei ausschließlicher Bewegung des Arbeitskolbens durch Druckgasbeaufschlagung im zweiten Arbeitsbereich;
- Figur 6a:: eine vergrößerte Ansicht des Bereichs F in Figur 6;

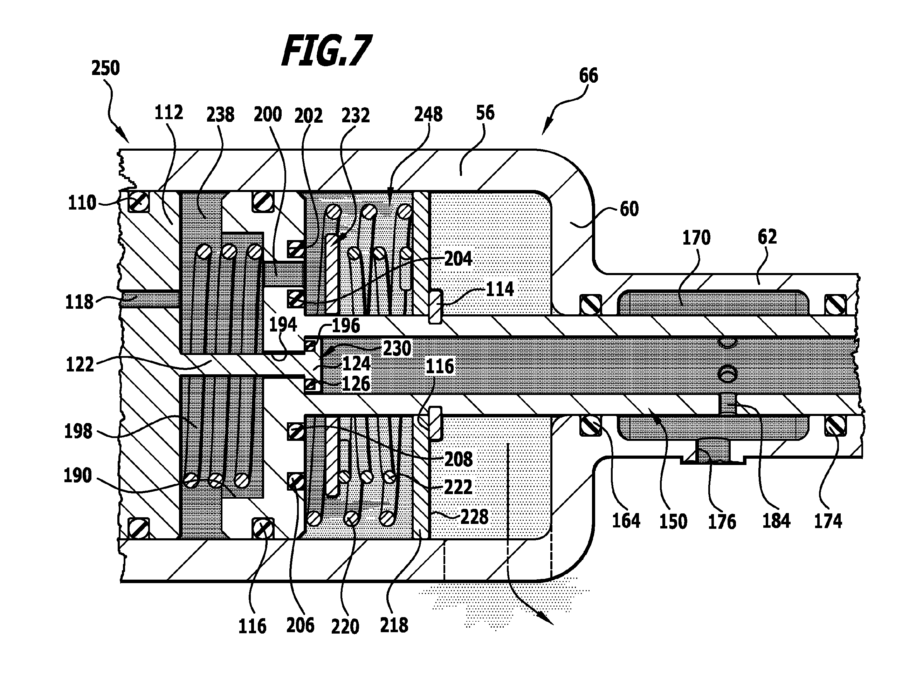

- Figur 7:: eine vergrößerte Ansicht der Regelungseinrichtung des Instruments beim Entlüften des Arbeitsraums;
- Figur 8a:: eine vergrößerte Ansicht des die Druckregelungseinrichtung umfassenden Teils einer alternativen Ausführungsform des Instruments in der Grund- oder Ausgangsstellung; und
- Figur 8b:: eine Ansicht entsprechend Figur 8a bei ausschließlicher Bewegung des Arbeitskolbens durch Druckgasbeaufschlagung im zweiten Arbeitsbereich.

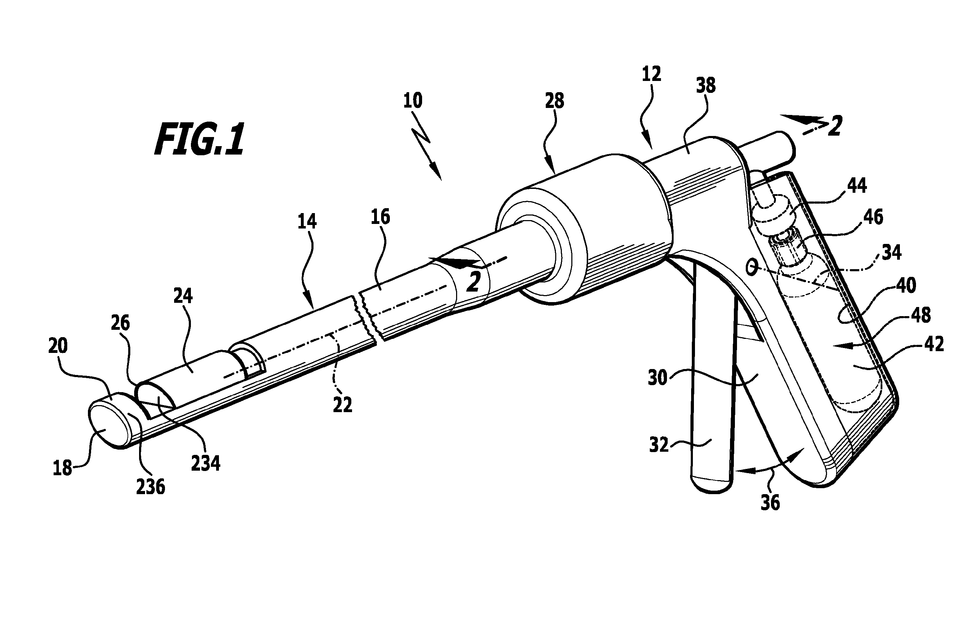

In Figur 1 ist schematisch ein chirurgisches Instrument dargestellt und insgesamt mit dem Bezugszeichen 10 versehen. Es ist in Form einer Knochenstanze ausgebildet und umfasst einen Handgriff 12 und ein mit dem Handgriff 12 verbundenes, optional auch lösbar verbindbares, Schaftteil 14 mit einem relativ zum Handgriff 12 feststehenden oder drehfest an diesem festlegbaren Schaft 16, an dessen distalem Ende 18 ein Ambossteil 20 in Form eines sich im Wesentlichen quer zu einer vom Schaft 16 definierten Längsachse 22 erstreckenden Vorsprungs ausgebildet ist, der als Widerlager für eine ein distales Ende eines im Schaft 16 verschiebbar gelagerten Stanzteils 24 bildende Schneide 26 bildet, welche zum Bearbeiten von Knochen und/oder Gewebe gegen das Ambossteil 20 gedrückt werden kann.

Der Handgriff 12, welcher auch ohne den Schaftteil 14 die Funktion eines erfindungsgemäßen chirurgischen Instruments ausüben kann, zum Beispiel als Implantatverriegelungsinstrument, um zwei Implantatteile relativ zueinander zu bewegen, umfasst einen im Wesentlichen rotationssymmetrisch ausgebildeten Antriebsteil 28, welcher sich bei mit dem Handgriff 12 verbundenem Schaftteil 14 in Verlängerung der Längsachse 22 erstreckt. An einem proximalen Ende des Antriebsteils 28 steht quer von diesem ein Griffbereich 30 ab, an welchem ein Betätigungselement 32 in Form eines um eine quer zur Längsachse 22 orientierte Schenkachse 34 verschwenkbaren Hebels verschwenkbar gelagert ist, welcher von einer Bedienperson in Richtung des Pfeils 36 auf den Griffbereich 30 hin verschwenkt werden kann.

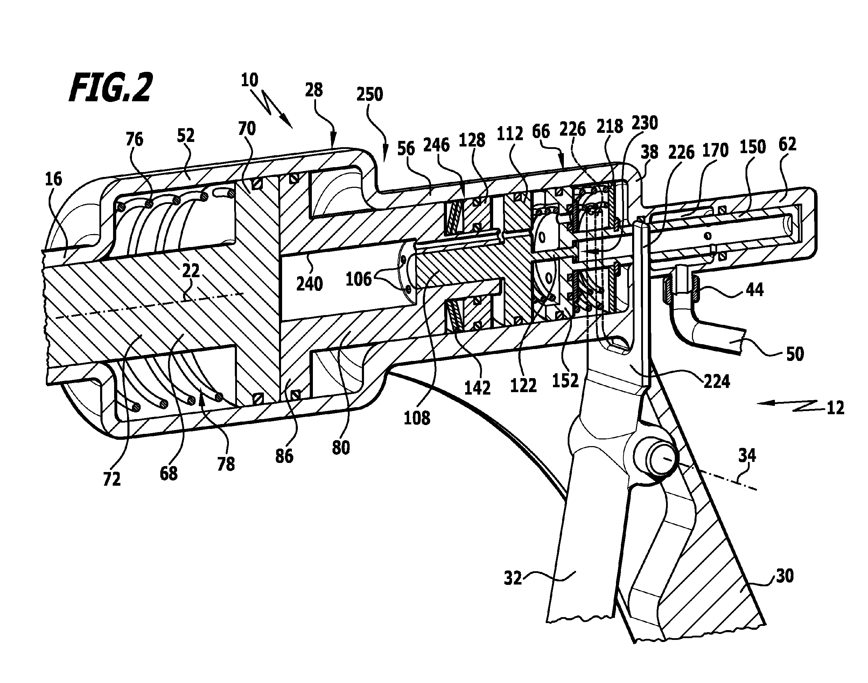

Der Griffbereich 30 bildet zusammen mit dem Antriebsteil 28 ein Gehäuse 38 aus, an welchem, wie ausschließlich schematisch in Figur 1 dargestellt, eine Druckgasbehälteraufnahme zum Aufnehmen eines Druckgasbehälters 42 ausgebildet ist. Die Druckgasbehälteraufnahme 40 ist in den Griffbereich 30 integriert. In ihr ist ein Druckgasanschluss 44 ausgebildet, der mit einem entsprechenden Anschlussstutzen 46 des eine Druckgasquelle 48 bildenden Druckgasbehälters 42 in Eingriff bringbar und gasdicht verbindbar ist. Optional wäre es möglich, den Griffbereich 30 schlanker auszubilden, wie beispielhaft in Figur 2 dargestellt, und ganz auf die Druckgasbehälteraufnahme 40 zu verzichten. Das Instrument 10 ist optional auch mit einer Druckgasquelle über einen Druckgasschlauch 50 verbindbar. Dies gestattet es, das Instrument 10 auch ohne Verwendung eines Druckgasbehälters 42 zu nutzen, indem es zum Beispiel mit einer Druckgasversorgung, wie sie üblicherweise in einem Krankenhaus vorhanden ist, verbunden wird.

Kernstück des Instruments 10 ist der Antriebsteil 28. Er umfasst einen sich proximalseitig an den Schaft 16 anschließenden Arbeitszylinder 52, dessen Innendurchmesser etwas mehr als doppelt so groß ist wie ein Innendurchmesser des Schafts 16. Über einen radial von der Längsachse 22 abstehenden Flansch 54 sind der Schaft 16 und der Arbeitszylinder 52 miteinander verbunden. Proximalseitig schließt sich an den Arbeitszylinder 52 ein Führungszylinder 56 an, welcher über einen weiteren, sich in radialer Richtung erstreckenden Flansch 58 mit dem Arbeitszylinder 52 verbunden ist. Ein Innendurchmesser des Führungszylinders 56 ist kleiner als ein Innendurchmesser des Arbeitszylinders 52, jedoch etwas größer als der Innendurchmesser des Schafts 16. An den Führungszylinder 56 schließt sich proximalseitig, über einen weiteren Flansch 60 verbunden, welcher sich in radialer Richtung von der Längsachse 22 weg erstreckt, ein Lagerzylinder 62 an, welcher proximalseitig mit einer Stirnwand 64 verschlossen ist. Der Innendurchmesser des Schafts 16 ist etwa zweieinhalb mal so groß wie der Innendurchmesser des Lagerzylinders 62. Der Arbeitszylinder 52, der Führungszylinder 56 und der Lagerzylinder 62 bilden einen Teil des Gehäuses 38 sowie eine insgesamt mit dem Bezugszeichen 250 bezeichnete Führungseinrichtung aus. Sie dienen zudem der Aufnahme des Antriebs des Instruments 10 sowie einer insgesamt mit dem Bezugszeichen 66 versehenen Regelungseinrichtung.

Im Arbeitszylinder 52 gehalten und geführt ist ein Arbeitskolben 68, welcher eine Kolbenscheibe 70 und einen einstückig mit dieser ausgebildeten und sich in distaler Richtung erstreckenden Zylinderabschnitt 72 umfasst. Der Außendurchmesser des massiven Zylinderabschnitts 72 entspricht dem Innendurchmesser des Schafts 16. Der Zylinderabschnitt 72 kann einstückig mit dem Stanzteil 24 ausgebildet sein. Optional kann der Zylinderabschnitt 72 auch mittels einer nicht dargestellten Kupplungseinrichtung vom Stanzteil 24 lösbar sein, um das Stanzteil 24 bei Bedarf auswechseln zu können. Ein Außendurchmesser der Kolbenscheibe 70 entspricht dem Innendurchmesser des Arbeitszylinders 52.

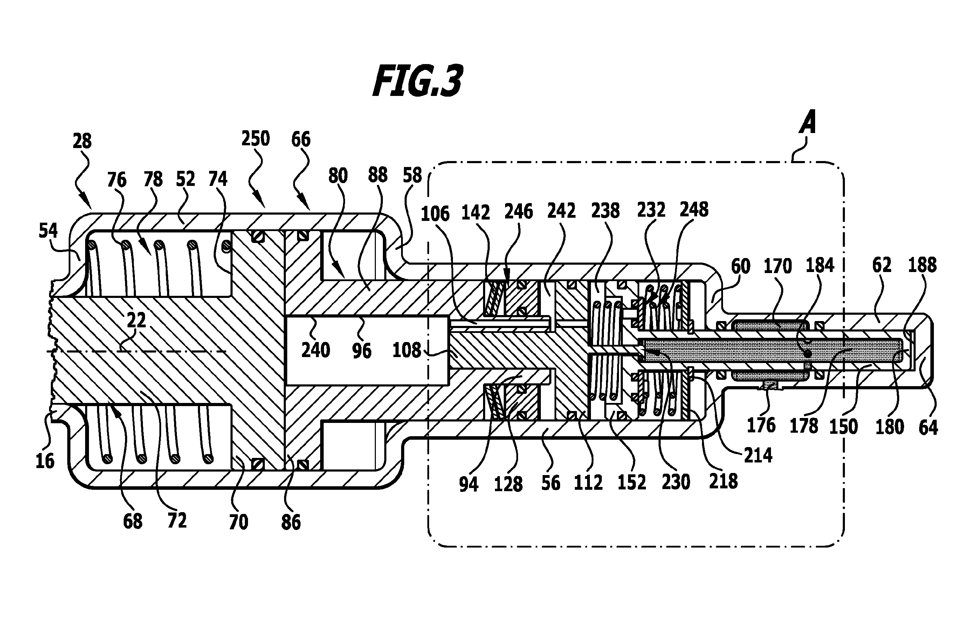

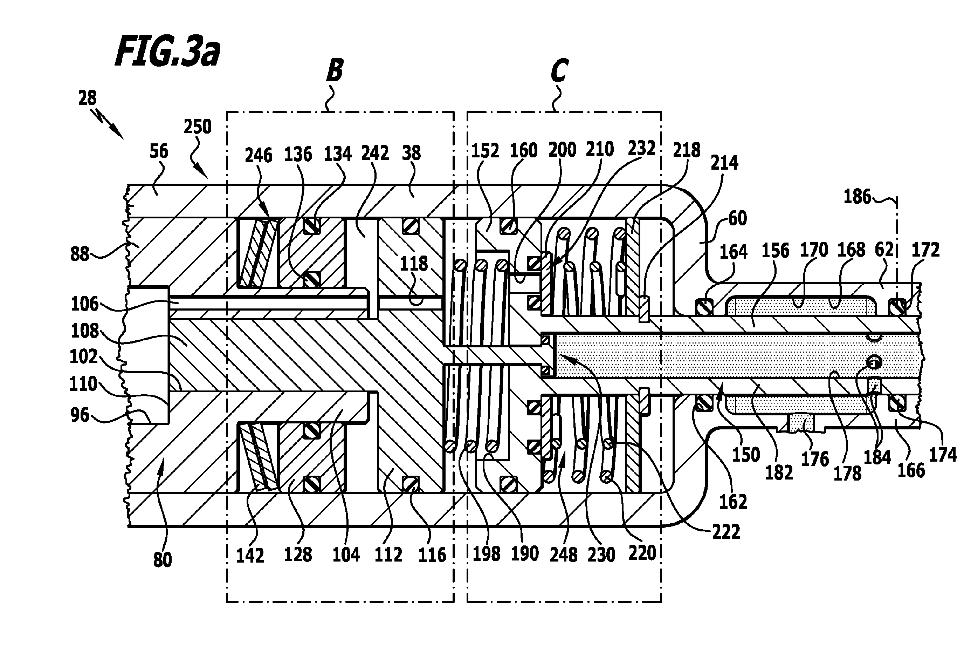

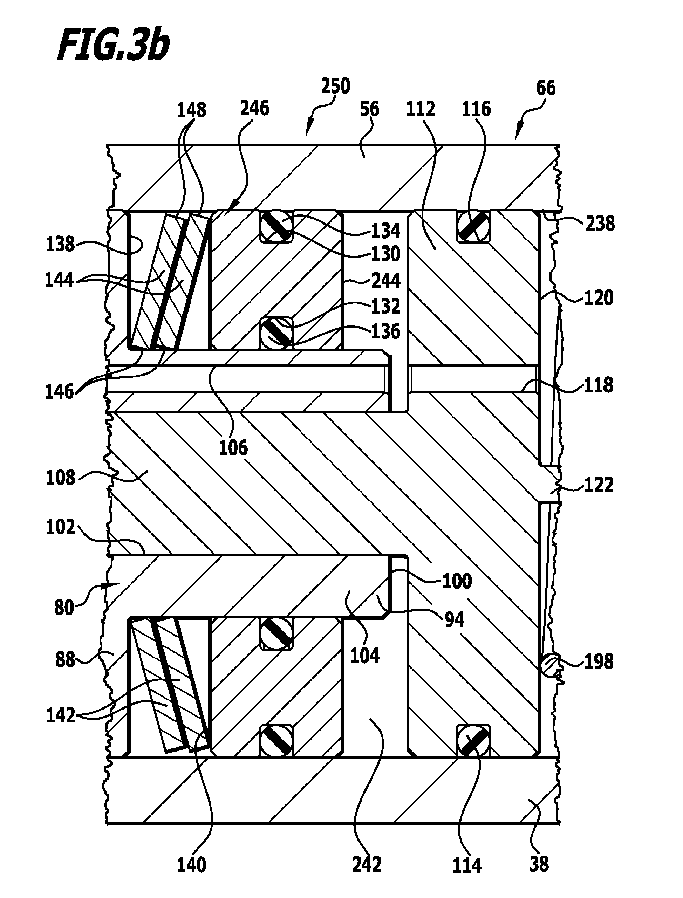

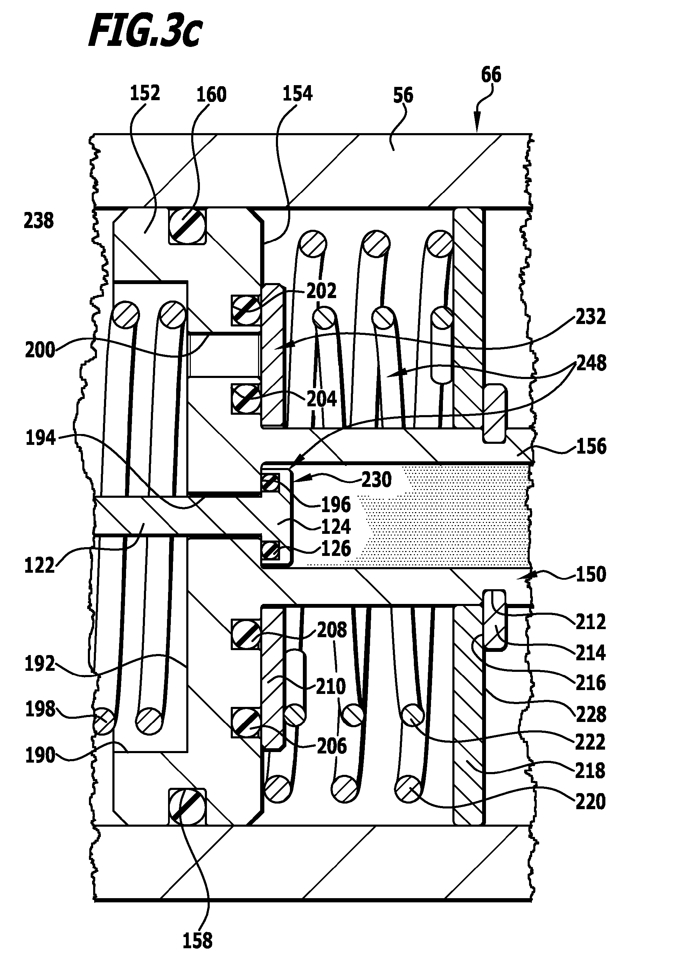

An einer in distaler Richtung weisenden Ringfläche 74 der Kolbenscheibe 70 stützt sich eine ein Rückstellglied 76 bildende Schraubenfeder ab. Distalseitig stützt sich das Rückstellglied 76 innen am Flansch 54 ab. Das Rückstellglied 76 ist als Druckglied ausgebildet und bildet einen Teil einer Rückstelleinrichtung 78, die den Arbeitskolben 68, wenn keine von einem Anwender eingeleiteten oder angeforderten Betätigungskräfte auf ihn wirken, in seine Grundstellung bewegt, nämlich in proximaler Richtung, bis er an einem Stützelement 80, wie in den Figuren 2 und 3 dargestellt, anliegt. Der Arbeitskolben 68 ist somit im Arbeitszylinder 52 und im Schaft 16, und damit zumindest teilweise durch die Führungseinrichtung 250, parallel zur Längsachse 22 in distaler und proximaler Richtung verschiebbar geführt. In eine auf einer von der Längsachse 22 weg weisenden Außenfläche ausgebildeten Ringnut 82 der Kolbenscheibe 70 ist ein Dichtelement 84 in Form eines O-Ringes eingesetzt zum Abdichten des Arbeitskolbens 68 und des Arbeitszylinders 52 relativ zueinander.

Das Stützelement 80 ist in Form eines Kolbens ausgebildet mit einer ein distales Ende des Stützelements 80 bildenden Kolbenscheibe 86, die einstückig mit einem sich in proximaler Richtung erstreckenden Zylinderabschnitt 88 ausgebildet ist. Ein Außendurchmesser der Kolbenscheibe 86 entspricht dem Innendurchmesser des Arbeitszylinders 52. Ein Außendurchmesser des Zylinderabschnitts 88 entspricht dem Innendurchmesser des Führungszylinders 56. In eine auf einer von der Längsachse 22 weg weisenden Außenfläche ausgebildeten Ringnut 90 der Kolbenscheibe 86 ist ein Dichtelement 92 in Form eines O-Rings eingesetzt zum Abdichten des Stützelements 80 und des Arbeitszylinders 52 relativ zueinander. Vom Zylinderabschnitt 88 steht proximalseitig ein kurzer hülsenförmiger Zylinderabschnitt 94 ab, welcher koaxial zur Längsachse 22 ausgerichtet ist. Ein Außendurchmesser des Zylinderabschnitts 94 ist etwa 1,5 mal größer als ein Innendurchmesser des Lagerzylinders 62.

Koaxial zur Längsachse 22 ist im Zylinderabschnitt 88 ein Sackloch 96 ausgebildet, welches in distaler Richtung weisend geöffnet ist. Der Innendurchmesser des Sacklochs 96 entspricht dem Außendurchmesser des Zylinderabschnitts 94 und ist damit kleiner als der Innendurchmesser des Schafts 16. Ein proximales Ende 100 des Zylinderabschnitts 94 ist mit dem Sackloch 96 über eine sich koaxial zur Längsachse 22 erstreckenden Bohrung 102 verbunden. In einer verbleibenden Zylinderwand 104 des Zylinderabschnitts 94 sind mehrere gleichmaßig über den Umfang desselben verteilte und sich parallel zur Längsachse 22 erstreckende Bohrungen 106 ausgebildet, die ebenfalls das Ende 100 mit dem Sackloch 96 verbinden.

In die Bohrung 102 eingesetzt ist ein Bolzenabschnitt 108, welcher distalseitig mit einem in distaler Richtung weisenden Boden 110 des Sacklochs 96 bündig abschließt. Der Bolzenabschnitt 108 ist etwas länger als die Bohrung 102 und proximalseitig einstückig mit einer weiteren Kolbenscheibe 112 verbunden, deren Außendurchmesser dem Innendurchmesser des Führungszylinders 96 entspricht. Die Kolbenscheibe 112 ist somit etwas vom proximalen Ende 100 des Zylinderabschnitts 94 beabstandet. In eine auf einer von der Längsachse 22 weg weisenden Außenfläche ausgebildeten Ringnut 114 der Kolbenscheibe 112 ist ein Dichtelement 116 in Form eines O-Rings eingesetzt, um die Kolbenscheibe 112 und den Führungszylinder 56 relativ zueinander abzudichten.

Koaxial zu den Bohrungen 106 sind an der Kolbenscheibe 112 in entsprechender Zahl und mit demselben Innendurchmesser Bohrungen 118 vorgesehen und fluchtend mit den Bohrungen 106 ausgerichtet. Der Bolzenabschnitt 108 kann in die Öffnung 102 eingeklebt sein. Optional kann das Sackloch 96 mit einem Innengewinde versehen sein, der Bolzenabschnitt 108 mit einem korrespondierenden Außengewinde, so dass der Bolzenabschnitt 108 und das Stützelement 80 miteinander verschraubbar sind. In jedem Fall ist der Bolzenabschnitt 108 unbeweglich mit dem Zylinderabschnitt 94 verbunden.

Von einer proximalen Endfläche 120 der Kolbenscheibe 112 steht ein zylindrischer Ventilstift 122 koaxial zur Längsachse 22 in proximaler Richtung weisend ab. An einem proximalen Ende desselben ist ein Ringflansch 124 ausgebildet, an welchem ein Dichtelement 126 gehalten ist, welches in eine in distaler Richtung weisende Ringnut eingesetzt ist.

Auf dem Zylinderabschnitt 94 parallel zur Längsachse 22 geführt ist ein Klemmkolben 128. Er weist zwei Ringnuten 130 und 132 auf, die in radialer Richtung von der Längsachse 22 weg beziehungsweise in radialer Richtung auf die Längsachse 22 hin weisend geöffnet sind. In die Ringnuten 130 und 132 eingesetzt sind Dichtelemente 134 und 136 in Form von O-Ringen zum Abdichten des Klemmkolbens 128 und des Führungszylinders 56 beziehungsweise des Klemmkolbens 128 und des Zylinderabschnitts 94 relativ zueinander.

Zwischen einer in proximaler Richtung weisenden Ringfläche 138 des Zylinderabschnitts 88 und einer in distaler Richtung weisenden Ringfläche 140 des Klemmkolbens 128 sind zwei identische Klemmelemente 142 in Form von Scheiben 144 angeordnet. Selbstverständlich können auch mehr als zwei Klemmelemente vorgesehen sein, die zudem nicht identisch ausgebildet sein müssen. Die Scheiben 144 definieren keine Ebene senkrecht zur Längsachse 22, sondern sind relativ zur Längsachse 22 etwas geneigt. Innenkanten 146 der Scheiben 144, die in radialer Richtung auf die Längsachse 22 hin weisen, sind so etwas distaler angeordnet als von der Längsachse 22 weg weisende Außenkanten 148 der Scheiben 144. Die distalere Scheibe 144 liegt vorzugsweise benachbart der Innenkante 146 an der Ringfläche 138 an, die proximale Scheibe 144 benachbart ihrer Außenkante 148 an der Ringfläche 140.

Die Regelungseinrichtung 66 umfasst ferner einen Druckregelkolben 150, der eine Kolbenscheibe 152 umfasst sowie einen von einer in proximaler Richtung weisenden Seitenfläche 154 der Kolbenscheibe 152 abstehenden Zylinderabschnitt 156. Die Kolbenscheibe 152 weist einen Außendurchmesser auf, der dem Innendurchmesser des Führungszylinders 56 entspricht. In eine in einer von der Längsachse 22 weg weisenden Außenfläche ausgebildeten Ringnut 158 der Kolbenscheibe 152 ist ein Dichtelement 160 in Form eines O-Rings eingesetzt, um die Kolbenscheibe 152 und den Führungszylinder 56 relativ zueinander abzudichten. Der Zylinderabschnitt 156 weist einen Außendurchmesser auf, der dem Innendurchmesser des Lagerzylinders 62 entspricht.

Zum Abdichten des Zylinderabschnitts 156 und des Führungszylinders 56 relativ zueinander ist am Führungszylinder 56 im Bereich des Flansches 58 eine in Richtung auf die Längsachse 22 hin geöffnete Ringnut 162 ausgebildet, in die ein Dichtelement 164 in Form eines O-Rings eingesetzt ist. Etwas beabstandet und proximalseitig der Ringnut 162 ist in einer den Lagerzylinder 62 ausbildenden Ringwand 166 eine sich in Richtung der Längsachse 22 erstreckende ringnutartige Ausnehmung 168 ausgebildet, die einen den Zylinderabschnitt 156 umgebenden Ringraum 170 definiert. Proximalseitig der Ausnehmung 168 und etwas beabstandet von dieser ist analog zur Ringnut 162 eine weitere Ringnut 172 ausgebildet, in die ein Dichtelement 174 in Form eines weiteren O-Rings eingesetzt ist zum Abdichten des Zylinderabschnitts 156 und des Lagerzylinders 62 relativ zueinander proximalseitig des Ringraums 170. Der Druckgasanschluss 44, welcher in Form eines hülsenförmigen Stutzens senkrecht zur Längsachse 22 außen vom Lagerzylinder 62 absteht, ist über eine Bohrung 176, deren Längsachse senkrecht zur Längsachse 22 orientiert ist, mit dem Ringraum 170 verbunden.

Der Zylinderabschnitt 156 ist hohl und definiert einen langgestreckten hohlzylindrischen Innenraum 178, welcher sich von einer proximalseitigen Stirnwand 180 des Zylinderabschnitts 156 bis zur Kolbenscheibe 152 erstreckt. Eine hülsenförmige verbleibende Wand 182 des Zylinderabschnitts 156 ist mit mehreren über den Umfang der Wand 182 verteilten Bohrungen 184 versehen, deren Längsachsen die Längsachse 22 senkrecht schneiden. Alle Bohrungen 184 sind in einer Ebene 186 angeordnet, die senkrecht zur Längsachse 22 orientiert ist. Die Bohrungen 184 sind ferner derart am Zylinderabschnitt 156 positioniert, dass sie mit dem Ringraum 170 in Fluidverbindung stehen, selbst dann, wenn die Stirnwand 180 an einer in distaler Richtung weisenden Stirnfläche 188 der Stirnwand 64 anliegt. Die Erstreckung, das heißt eine Länge, des Ringraums 170 parallel zur Längsachse 122 ist so gewählt, dass die Bohrungen 184 mit dem Ringraum 170 auch dann noch in Fluidverbindung stehen, wenn der Zylinderabschnitt 156 seine distalste Stellung einnimmt, also maximal weit in distaler Richtung verschoben ist. So ist es möglich, dass unabhängig von einer Stellung des Zylinderabschnitts 156 relativ zum Lagerzylinder 62 Druckgas von der Druckgasquelle 48 über den Druckgasanschluss 44 in den Innenraum 178 gelangen kann.

In der Kolbenscheibe 152 ist ein in distaler Richtung weisendes flaches Sackloch 190 ausgebildet. Einen Boden 192 des Sacklochs 190 durchsetzt eine koaxial zur Längsachse 22 ausgebildete Bohrung 194, deren Innendurchmesser etwas größer ist als ein Außendurchmesser des Ventilstifts 122, der die Bohrung 194 durchsetzt. Ein Innendurchmesser des Innenraums 178 ist etwas größer als ein Innendurchmesser der Bohrung 194. Ein Außendurchmesser des Ringflansches 124 ist etwas kleiner als ein Innendurchmesser des Innenraums 178. Der Ringflansch 124 kann mit der Seitenfläche 154 in Anlage gebracht werden, und zwar mit einem Teil derselben, nämlich einer in proximaler Richtung weisenden Ringfläche 196, die den Innenraum 178 die Bohrung 194 umgebend begrenzt. Mit dem Dichtelement 126 können der Ringflansch 124 und die Kolbenscheibe 152 relativ zueinander abgedichtet werden.

Ein erstes Druckglied 198 in Form einer Schraubenfeder stützt sich distalseitig an der Endfläche 120 und proximalseitig am Boden 192 ab und drückt die Kolbenscheibe 152 in proximaler Richtung gegen den Ringflansch 124. Die Bohrung 194 ist dann geschlossen.

An der Kolbenscheibe 152 ist parallel zur Bohrung 194, jedoch von dieser in radialer Richtung beabstandet, eine weitere Bohrung 200 vorgesehen, die den Boden 192 und die Seitenfläche 154 miteinander verbindet. Konzentrisch zur Längsachse 22 sind in der Seitenfläche 154 zwei Ringnuten 202 und 204 ausgebildet, die in proximaler Richtung weisend geöffnet sind. Sie sind derart angeordnet, dass die Ringnut 202 in radialer Richtung von der Längsachse 22 etwas weiter beabstandet ist als die Bohrung 200, die Ringnut 204 etwas näher zur Längsachse 22 angeordnet ist als die Bohrung 200. In die Ringnuten 202 und 204 sind jeweils Dichtelemente 206 und 208 in Form von O-Ringen eingesetzt, die mit einer den Zylinderabschnitt 156 umgebenden Ventilscheibe 210 zusammenwirken, um die Bohrung 200 gasdicht zu verschließen. Die Ventilscheibe 200 definiert eine Ebene senkrecht zur Längsachse 22.

Auf einer Außenseite des Zylinderabschnitts 156 ist eine flache schmale Ringnut 212 ausgebildet, und zwar in einem Abstand zur Seitenfläche 154, der etwas kleiner ist als eine Erstreckung des Ringraums 170 parallel zur Längsachse. In die Ringnut 212 eingesetzt ist eine einen Außendurchmesser, der kleiner als ein Außendurchmesser der Lagerhülse 62 ist, aufweisende Scheibe 214, welche eine in distaler Richtung weisende ringförmige Anschlagfläche 216 definiert. Die Scheibe 214 kann gleichzeitig als Anschlag dienen, um eine Bewegung des Zylinderabschnitts 156 in proximaler Richtung zu begrenzen, nämlich dann, wenn die Scheibe 214 am Flansch 60 anschlägt. Zwischen der Scheibe 214 und der Seitenfläche 154 ist ein Betätigungsglied 218 in Form einer flachen Scheibe verschiebbar auf dem Zylinderabschnitt 156 angeordnet, deren Innendurchmesser an den Außendurchmesser des Zylinderabschnitts 156 angepasst ist und deren Außendurchmesser an den Innendurchmesser des Führungszylinders 56 angepasst ist. Ein zweites Druckglied 220 in Form einer Schraubenfeder stützt sich proximalseitig am Betätigungsglied 218 und distalseitig an der Seitenfläche 154 ab. Ein Durchmesser des zweiten Druckglieds 220 ist etwas größer als ein Außendurchmesser der Ventilscheibe 210.

Ein drittes Druckglied 222 stützt sich proximalseitig ebenfalls am Betätigungsglied 218 ab, distalseitig jedoch an der Ventilscheibe 210. Eine Federkonstante des zweiten Druckglieds 220 ist etwas größer als eine Federkonstante des dritten Druckglieds 222. Das zweite Druckglied 220 drückt das Betätigungsglied 218 gegen die Anschlagfläche 216. Das dritte Druckglied 222 drückt die Ventilscheibe 210 gegen die Dichtelemente 206 und 208 und verschließt so die Bohrung 200.

Das Betätigungselement 32 erstreckt sich über die Schwenkachse 34 hinaus und definiert einen Hebelarm 224, welcher gabelförmig und zwei parallel zueinander verlaufende Schenkel 226 umfassend ausgebildet ist. Er ist so angeordnet, dass die Schenkel 226 des gabelförmigen Hebelarms 224 direkt am Betätigungsglied 218 auf dessen in proximaler Richtung weisenden Seitenfläche 228 anliegen. Wird das Betätigungsglied 32 in Richtung auf den Griffbereich 30 um die Schwenkachse 34 hin verschwenkt, so werden die Schenkel 226 in distaler Richtung bewegt, nehmen das Betätigungsglied 218 mit und verschieben es in distaler Richtung.

Die Funktionsweise des Instruments 10, insbesondere auch die Funktionsweise der Regelungseinrichtung 66, wird nachfolgend anhand der Figuren erläutert.

In den Figuren 1 bis 3a ist das Instrument 10 in seiner Grundstellung dargestellt. Das Betätigungselement 32 ist dabei unbetätigt. Die Druckgasquelle 48 steht mit dem Innenraum 178 in Fluidverbindung. Der von der Druckgasquelle 48 bereitgestellte Arbeitsdruck des Druckgases wirkt auf den Ringflansch 124 und drückt diesen gegen die Ringfläche 196. Ein Ventil 230, das definiert wird durch die Bohrung 194 in Verbindung mit dem Ringflansch 124 und dem Dichtelement 126, nimmt in der Grundstellung des Instruments 10 eine geschlossene Stellung ein. Das Druckgas kann somit nicht durch die Bohrung 194 hindurchströmen. Das Rückstellglied 76 drückt den Arbeitskolben 68 in proximaler Richtung. Die Kolbenscheibe 70 liegt an der Kolbenscheibe 86 an, welche bis an den Flansch 58 heranbewegt wird. Die Kolbenscheibe 112, die fest mit der Kolbenscheibe 86 verbunden ist, wird somit zwangsweise ebenfalls in proximaler Richtung bewegt. Das erste Druckglied 198 drückt die Kolbenscheibe 152 in proximaler Richtung ebenfalls gegen den Ringflansch 124. Das zweite Druckglied 220 drückt das Betätigungsglied 218 in proximaler Richtung gegen die Anschlagfläche 216 und das dritte Druckglied 222 drückt die Ventilscheibe 210 gegen die Dichtelemente 204 und 206 und verschließt die Bohrung 200. Die Bohrung 200 in Verbindung mit der Ventilscheibe 210 und den Dichtelementen 206 und 208 definiert ein Entlüftungsventil 230, welches nur entgegen der Wirkung des dritten Druckglieds 222 geöffnet werden kann. Das Ventil 230 kann nur entgegen der Wirkung des ersten Druckglieds 198 geöffnet werden.

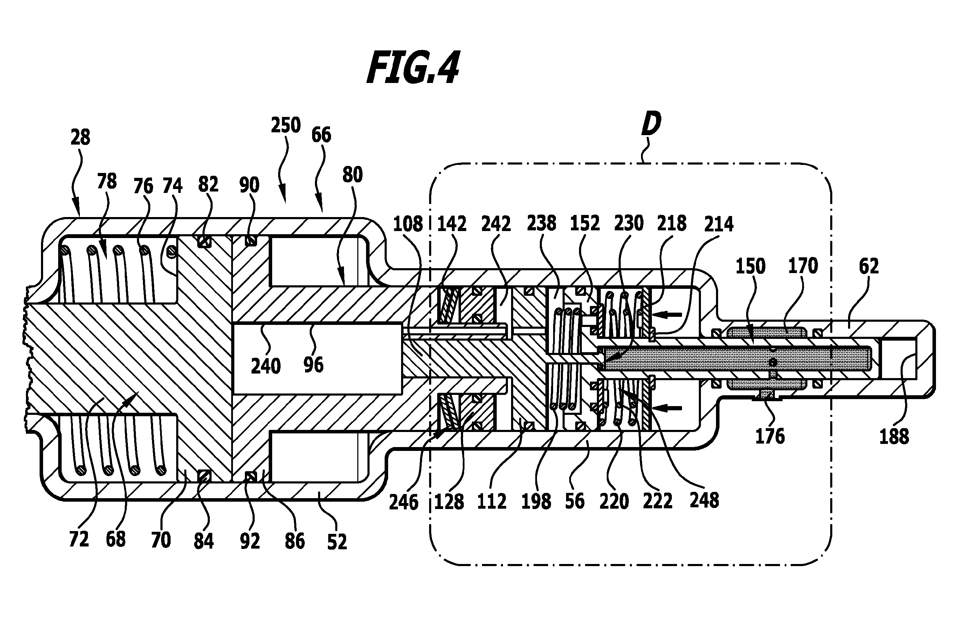

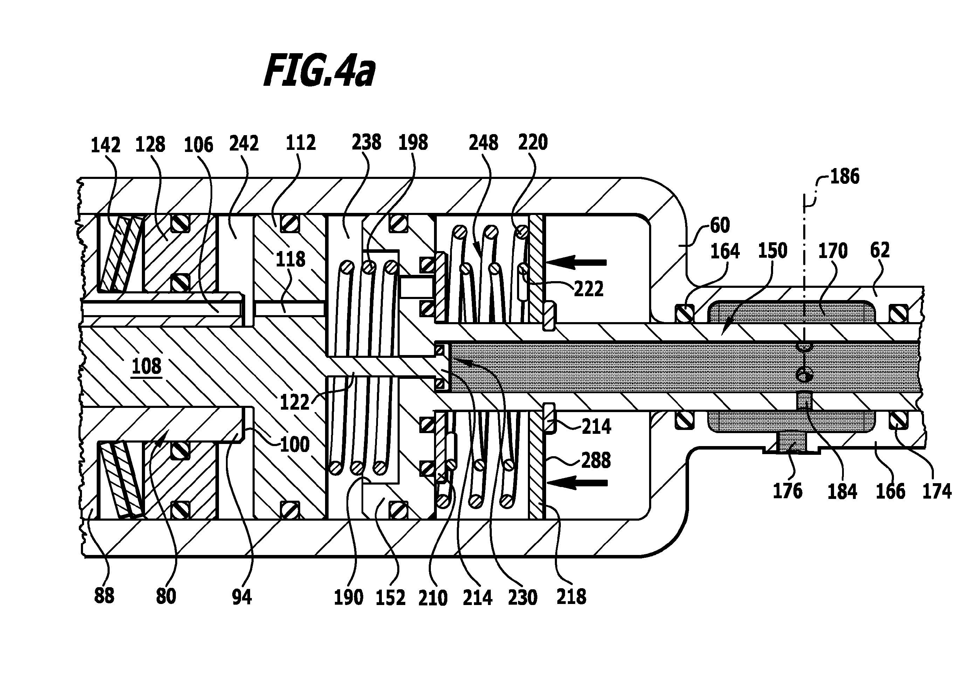

Die vom ersten Druckglied 198 ausgeübte Kraft ist geringfügig größer als die Summe der vom zweiten und dritten Druckglied 220, 222 ausgeübten Kräfte. Wirken dem Arbeitskolben 68 nur geringe Kräfte im Bereich der Werkzeugelemente 234 und 236 definierenden Schneide 26 beziehungsweise des Ambossteils 20 entgegen, so wird in Folge einer Verschwenkung des Betätigungselements 32 in Richtung auf den Griffbereich 30 hin das Betätigungsglied 218 in distaler Richtung bewegt. In diesem ersten oder mechanischen Arbeitsbereich des Instruments 10 erfolgt eine Bewegung des Arbeitskolbens 68 allein aufgrund der mit dem Betätigungselement 32 aufgebrachten Vorschubkraft in distaler Richtung. Die eingeleitete Betätigungskraft wird über das zweite Druckglied 220 und das dritte Druckglied 222 auf die Kolbenscheibe 152 übertragen und mittels der ersten Druckglieds 198 auf die Kolbenscheibe 112. Der sich direkt an der Kolbenscheibe 86 abstützende Arbeitskolben 68 wird so ohne Unterstützung von Druckgas in distaler Richtung bewegt. Der erste Arbeitsbereich des Instruments 10 ist in den Figuren 4 und 4a dargestellt.

Steigt der Widerstand zwischen den Werkzeugelementen 234 und 236 an und übersteigt die dort wirkende Kraft die Summe der vom zweiten Druckglied 220 und vom dritten Druckglied 222 ausgeübten Kräfte, so werden die Druckglieder 220 und 222 komprimiert. Auch das erste Druckglied 198 wird komprimiert, was zur Folge hat, dass das Ventil 230 öffnet und Druckgas vom Innenraum 178 in den zwischen den Kolbenscheiben 112 und 152 definierten Ringraum 238, in dem das erste Druckglied 198 angeordnet ist, einströmen kann. Das Druckgas kann so lange nachströmen bis sich wieder ein Kräftegleichgewicht zwischen der Summe der vom ersten Druckglied 198 ausgeübten Kraft und der durch das Druckgas im Ringraum 238 distalseitig der Kolbenscheibe 152 anliegenden Kraft und der Summe der von den Druckgliedern 220 und 222 an der anderen Seite der Kolbenscheibe 152 ausgeübten Kräfte eingestellt hat. Durch Verschwenken des Betätigungselements 32 auf den Griffbereich 30 hin wird somit sukzessive Druckgas in den Ringraum 238 eingeleitet. Der Arbeitskolben 68 liegt aber noch immer am Stützelement 80 an.

Der Ringraum 238 steht über die Bohrungen 118 und die Bohrungen 106 mit einem vom Sackloch 96 definierten Arbeitsraum 240 in Fluidverbindung. Der herrschende Gasdruck liegt jedoch auch in einem Ringraum 242 zwischen dem Klemmkolben 128 und der Kolbenscheibe 112 an und wirkt auf eine in proximaler Richtung weisende Ringfläche 244 des Klemmkolbens 128.

Ab einem bestimmten Gasdruck im Instrument 10 wird eine insgesamt mit dem Bezugszeichen 246 versehende Feststelleinrichtung aktiviert. Sie umfasst den Klemmkolben 128 und die Klemmelemente 142. Die Kolbenscheibe 112 verschiebt sich dabei in Richtung auf den Klemmkolben 128 hin, und mit Unterstützung des im Druckgas herrschenden Drucks wird der Klemmkolben 128 in distaler Richtung bewegt und drückt die an ihm anliegende Außenseite der Scheibe 144 in distaler Richtung, wodurch das Stützelement 80 und der Führungszylinder 56 gegeneinander verklemmt werden. Der Arbeitskolben 68 stützt sich in der beschriebenen Weise an dem mittels der Feststelleinrichtung 246 am Führungszylinder 56 festgelegten Stützelement 80 ab, welches so von einer Bewegung des Betätigungsglieds 218 entkoppelt ist. Darüber hinaus steigt eine zwischen dem Stützelement 80 und dem Führungszylinder 56 wirkende Klemmkraft mit zunehmendem Arbeitsdruck an.

Bei einer weiteren Bewegung des Betätigungselements 32 in Richtung auf den Griffbereich 30 hin werden die Druckglieder 220 und 222 weiter verkürzt, so dass sich der im Arbeitsraum 240 herrschende Druck bis zu einem Maximum erhöhen kann. Dieser herrschende Druck bewirkt die gewünschte hohe Kraft zwischen den Werkzeugelementen 234 und 236, indem das Druckgas im Arbeitsraum 240 den Arbeitskolben 68 in distaler Richtung drückt.

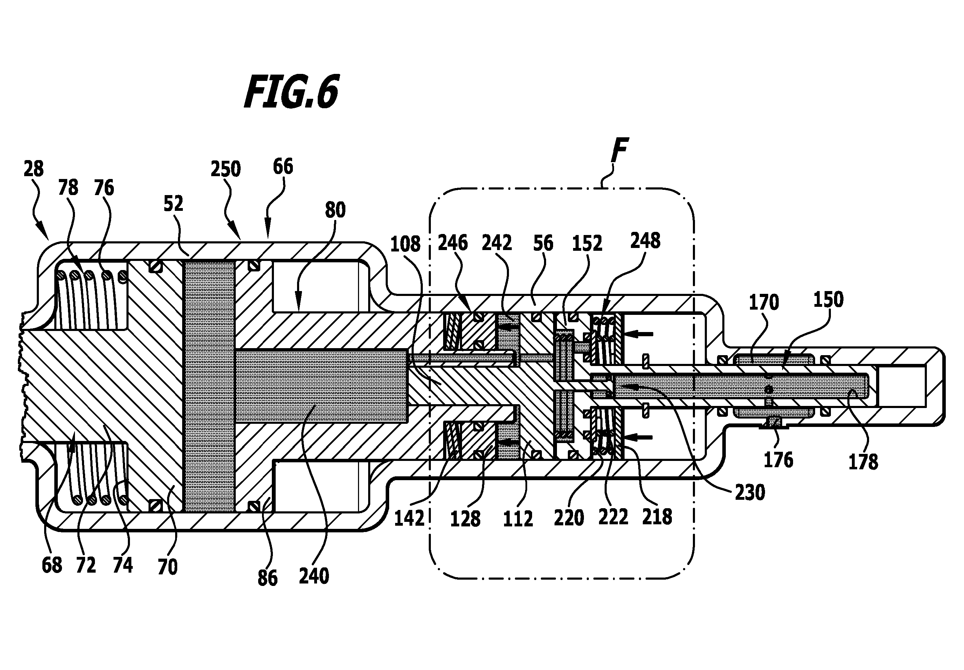

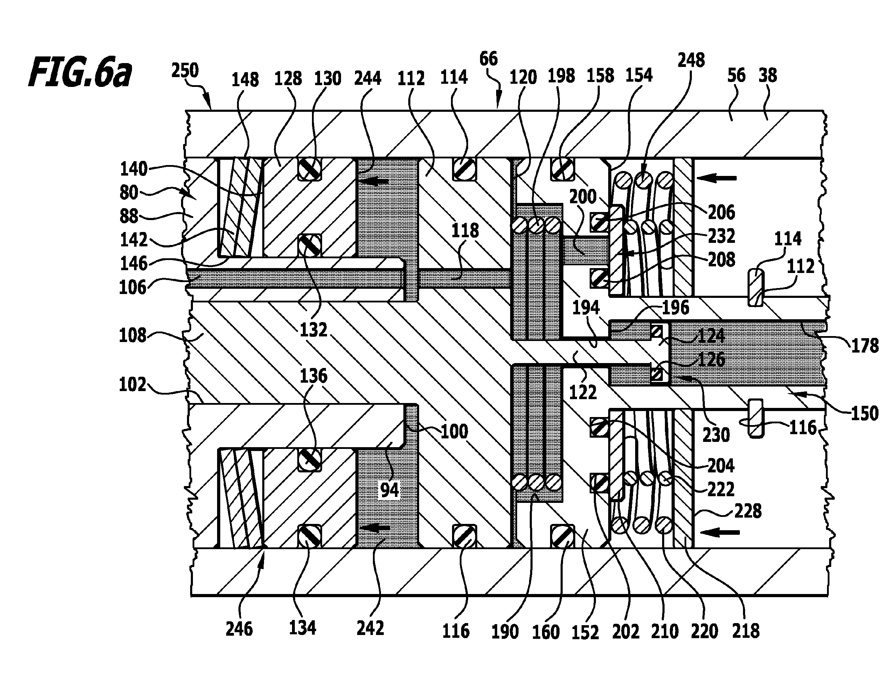

Wie beschrieben arbeitet das Instrument 10 unterhalb einer vorgegebenen Grenzkraft, die eingestellt werden kann durch entsprechende Auswahl der Druckglieder 198, 220 und 222, im ersten oder mechanischen Arbeitsbereich. Erst oberhalb der Grenzkraft wird durch die Regelungseinrichtung 66 ein zweiter oder pneumatischer Arbeitsbereich definiert, in welchem der Arbeitskolben 68 nur noch durch Beaufschlagen mit Druckgas in distaler Richtung bewegt wird. Dieser zweite Arbeitsbereich ist in den Figuren 6 und 6a dargestellt.

Mit dem Instrument 10 kann so eine gewünschte Maximalkraft allein durch das Druckgas aufgebracht werden. Um insbesondere bei der Verwendung eines Druckgasbehälters 42 einen Gasverbrauch auf ein Minimum zu reduzieren, wird das Druckgas durch die Regelungseinrichtung 66 erst bei hohen wirkenden Kräften zugeschaltet.

Ein Druckregler 248 des Instruments wird gebildet durch das Ventil 230 und die drei Druckglieder 198, 220 und 222 in Verbindung mit der Kolbenscheibe 152. Er stellt sicher, dass ein Arbeitsdruck des Druckgases proportional zu einer auf das Betätigungselement 32 von einer Bedienperson ausgeübten Kraft ist. Damit lässt sich die vom Arbeitskolben 68 ausgeübte Kraft stufenlos erhöhen, wobei dem Anwender so eine größtmögliche Taktilität zur Verfügung steht. Da auch im zweiten oder pneumatischen Arbeitsbereich bedingt durch eine Verkürzung der Druckglieder 220 und 222 ein definierter Weg sowohl des Betätigungselements 32 als auch des Betätigungsglieds 218 zurückgelegt wird, wird auch die Taktilität für den Anwender im zweiten Arbeitsbereich verbessert.

Bei einer Vorwärtsbewegung des Betätigungsglieds 218 ist die Kraft, die das dritte Druckglied 222 ausübt, stets geringfügig größer als die auf die Ventilscheibe 210 wirkende Druckkraft, so dass das Druckgas nicht entweichen kann, insbesondere nicht aus dem Arbeitsraum 240.

Wird die Kraft auf das Betätigungselement 32 vom Anwender reduziert und dieses vom Griffbereich 30 weg verschwenkt, werden beim Rückhub des Arbeitskolbens 68 die Druckglieder 220 und 222 verlängert, so dass die vom dritten Druckglied 222 ausgeübte Kraft nicht mehr ausreicht, um die Bohrung 200 zu verschließen. Der Druck im Instrument 10 fällt ab, indem das Druckgas über das Entlüftungsventil 232 entweicht, bis die Kraft des dritten Druckglieds 222 wieder ausreicht, um die Bohrung 200 zu verschließen. Auf diese Weise fällt der Druck im Arbeitsraum 240 proportional zum zurückgelegten Weg des Betätigungsglieds 218 in proximaler Richtung ab, wenn auch mit einer gewissen Rücksteuerhysterese.

Um das Instrument 10 vor Überdruck zu schützen, kann zusätzlich, also optional, ein in den Figuren nicht dargestelltes Überdruckventil vorgesehen sein, insbesondere auch bei allen beschriebenen bevorzugten Ausführungsformen eines chirurgischen Instruments, welches in Fluidverbindung mit dem Innenraum 178 steht.

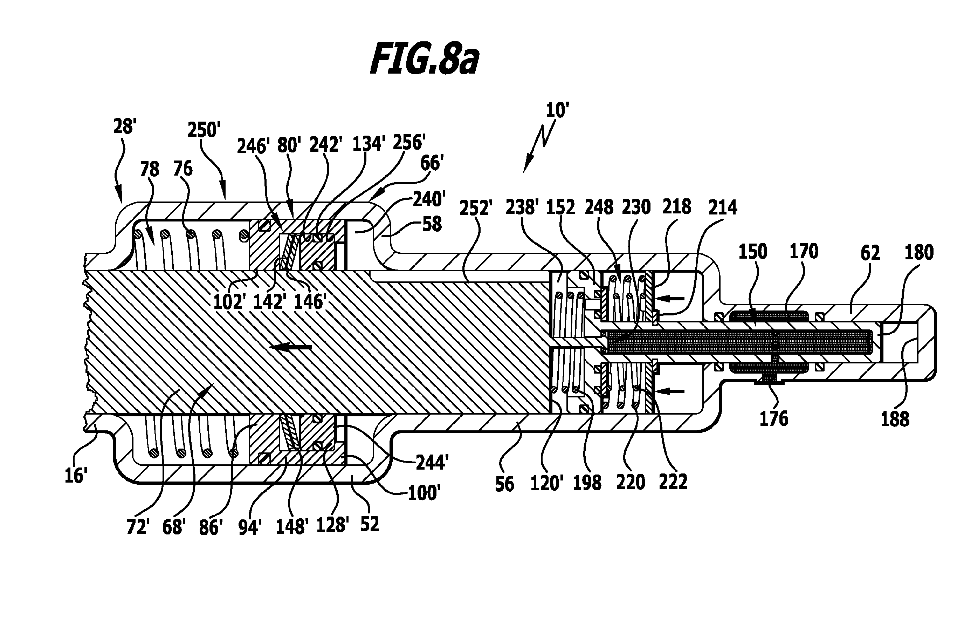

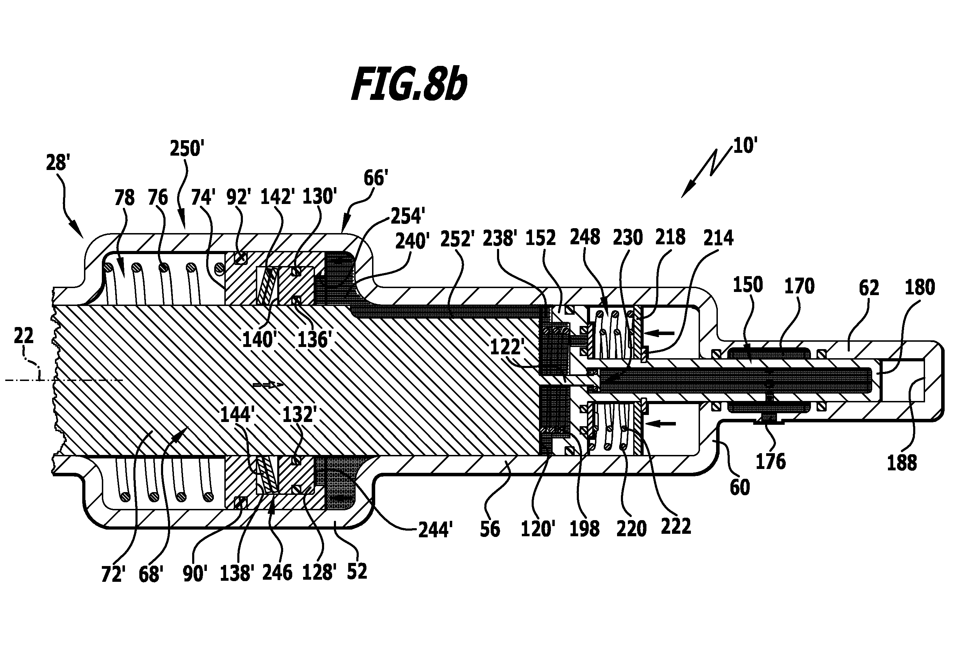

In den Figuren 8a und 8b ist schematisch ein gegenüber dem chirurgischen Instrument 10 teilweise modifiziertes Instrument in Form einer Knochenstanze dargestellt und insgesamt mit dem Bezugszeichen 10' versehen. Nachfolgend werden im Wesentlichen nur diejenigen Elemente und Bauteile des Instruments 10' sowie seine Funktionsweise beschrieben, soweit sie von denen des Instruments 10 abweichen. Ferner sei angemerkt, dass identische Teile mit denselben Bezugszeichen bezeichnet sind, bei ähnlichen beziehungsweise in ihrer Funktion ähnlichen oder einander entsprechenden Teilen dem jeweiligen Bezugszeichen ein Anstrich (" ' ") nachgestellt ist.

Kernstück des Instruments 10' ist der Antriebsteil 28', welcher gegenüber dem Antriebsteil 28 etwas modifiziert ist. Der Arbeitszylinder 52, der Führungszylinder 56 und der Lagerzylinder 62 bilden einen Teil des Gehäuses 38 sowie eine insgesamt mit dem Bezugszeichen 250' bezeichnete Führungseinrichtung aus. Sie dienen zudem der Aufnahme des Antriebs des Instruments 10' sowie einer insgesamt mit dem Bezugszeichen 66' versehenen Regelungseinrichtung, welche gegenüber der Regelungseinrichtung 66 etwas modifiziert ist.

Im Arbeitszylinder 52 gehalten und geführt ist ein Arbeitskolben 68', welcher einen sich in distaler Richtung erstreckenden Zylinderabschnitt 72' umfasst. Der Außendurchmesser des massiven Zylinderabschnitts 72' entspricht dem Innendurchmesser des Schafts 16', welcher denselben Innendurchmesser wie der Führungszylinder 56 aufweist. Der Zylinderabschnitt 72' kann einstückig mit dem Stanzteil 24 ausgebildet sein. Optional kann der Zylinderabschnitt 72' auch mittels einer nicht dargestellten Kupplungseinrichtung vom Stanzteil 24 lösbar sein, um das Stanzteil 24 bei Bedarf auswechseln zu können. Von einer proximalen Endfläche 120' des Zylinderabschnitts 72' steht ein zylindrischer Ventilstift 122' koaxial zur Längsachse 22 in proximaler Richtung weisend ab. Der Ventilstift 122' stimmt in seiner Ausgestaltung und Funktion identisch mit dem Ventilstift 122 überein.

Der Zylinderabschnitt 72' weist eine sich parallel zur Längsachse 22 verlaufende Längsnut 252' auf, welche sich ausgehend von der Endfläche 120' in distaler Richtung erstreckt und einen Länge aufweist, welche etwas kleiner ist als eine Erstreckung des Arbeitszylinders 52 parallel zur Längsachse 22. Damit ergibt sich beim Instrument 10' insgesamt eine Konstruktion, bei der der Zylinderabschnitt 72' praktisch den Zylinderabschnitt 72 sowie das Stützelement des Instruments 10 ersetzt.

Das Instrument 10' umfasst ebenfalls eine Feststelleinrichtung 246', welche jedoch nicht im Bereich des Führungszylinders 56, sondern den Zylinderabschnitt 72' im Bereich des Arbeitszylinders 52 umgebend ausgebildet ist. Die Feststelleinrichtung 246' umfasst ein in Form eines Kolbens ausgebildetes Stützelement 80', welches eine Kolbenscheibe 86' umfasst. Diese ist koaxial zur Längsachse 22 mit einer Bohrung 102' versehen. Ein Außendurchmesser der Kolbenscheibe 86' entspricht einem Innendurchmesser des Arbeitszylinders 52. In eine auf einer von der Längsachse 22 weg weisenden Außenfläche ausgebildeten Ringnut 90' der Kolbenscheibe 86' ist ein Dichtelement 92' in Form eines O-Rings eingesetzt zum Abdichten des Stützelements 80' und des Arbeitszylinders 52 relativ zueinander.

Von der Kolbenscheibe 86' steht proximalseitig ein kurzer, hülsenförmiger und einstückig mit ihr ausgebildeter Zylinderabschnitt 94' ab, welcher koaxial zur Längsachse 22 ausgerichtet ist. Ein Außendurchmesser des Zylinderabschnitts 94' entspricht dem Außendurchmesser der Kolbenscheibe 86' und definiert zusammen mit einer von der Längsachse weg weisenden Außenfläche derselben eine gemeinsame Zylinderfläche. An einem proximalen Ende 100' des Zylinderabschnitts 94' steht in radialer Richtung auf die Längsachse 22 hin weisend ein kurzer Ringflansch 254' ab, welcher eine ringförmige und in distaler Richtung weisende Anschlagfläche 256' definiert. Das Stützelement 80' und der Zylinderabschnitt 72' begrenzen somit einen in proximaler Richtung offenen Ringraum 242'.

Auf dem Zylinderabschnitt 72' parallel zur Längsachse 22 geführt ist ein im Wesentlichen scheibenförmiger Klemmkolben 128'. Er weist zwei Ringnuten 130' und 132' auf, die koaxial zur Längsachse 22 ausgebildeten Zylinderflächen des Klemmkolbens 128' in radialer Richtung von der Längsachse 22 weg beziehungsweise in radialer Richtung auf die Längsachse 22 hin weisend geöffnet sind. In die Ringnuten 130' und 132' eingesetzt sind Dichtelemente 134' und 136' in Form von O-Ringen zum Abdichten des Klemmkolbens 128' und des Arbeitszylinders 52 beziehungsweise des Klemmkolbens 128' und des Zylinderabschnitts 72' relativ zueinander.

Zwischen einer in proximaler Richtung weisenden und den Ringraum 242' distalseitig begrenzenden Ringfläche 138' der Kolbenscheibe 86' und einer in distaler Richtung weisenden Ringfläche 140' des Klemmkolbens 128' sind zwei identische Klemmelemente 142' in Form von Scheiben 144' angeordnet. Selbstverständlich können auch mehr als zwei Klemmelemente vorgesehen sein, die zudem nicht identisch ausgebildet sein müssen. Die Scheiben 144' definieren keine Ebene senkrecht zur Längsachse 22, sondern sind relativ zur Längsachse 22 etwas geneigt. Innenkanten 146' der Scheiben 144', die in radialer Richtung auf die Längsachse 22 hin weisen, sind so etwas distaler angeordnet als von der Längsachse 22 weg weisende Außenkanten 148' der Scheiben 144'. Die distalere Scheibe 144' liegt vorzugsweise benachbart der Innenkante 146' an der Ringfläche 138' an, die proximale Scheibe 144 benachbart ihrer Außenkante 148' an der Ringfläche 140'. Ein Abstand zwischen der Anschlagfläche 256' und der Ringfläche 138' ist so gewählt, dass eine in proximaler Richtung weisende Ringfläche 244' des Klemmkolbens 128' an der Anschlagfläche 256' anliegt. Die Klemmelemente 142' halten den Klemmkolben 128' unter Vorspannung in Anlage am Ringflansch 254'.

An einer in distaler Richtung weisenden Ringfläche 74' der Kolbenscheibe 86' stützt sich die ein Rückstellglied 76 bildende Schraubenfeder ab. Distalseitig stützt sich das Rückstellglied 76 innen am Flansch 54 ab. Das Rückstellglied 76 ist als Druckglied ausgebildet und bildet einen Teil der Rückstelleinrichtung 78, die den Arbeitskolben 68', wenn keine von einem Anwender eingeleiteten oder angeforderten Betätigungskräfte auf ihn wirken, in seine Grundstellung bewegt, nämlich in proximaler Richtung, bis er, falls das Rückstellglied 76 eine entsprechende Länge aufweist, im Extremfall am Flansch 58 anliegt. Ist das Rückstellglied 76 etwa nur halb so lang wie der Arbeitszylinder 52, erreicht das Stützelement 80' den Flansch 58 nicht. Dies ist schematisch in Figur 8a dargestellt. Vorzugsweise ist das Rückstellglied 76 jedoch eine ausreichende Länge auf, so dass das Stützelement 80' in der Grundstellung am Flansch 58 anliegt. Der Arbeitskolben 68' ist somit im Führungszylinder 56 und im Schaft 16', und damit zumindest teilweise durch die Führungseinrichtung 250', parallel zur Längsachse 22 in distaler und proximaler Richtung verschiebbar geführt. Optional kann ferner ein weiteres, in den Figuren 8a und 8b nicht dargestelltes Rückstellglied vorgesehen sein, welches sich einerseits innen am Flansch 54 und andererseits direkt am Arbeitskolben 68' abstützt, beispielsweise an einem in radialer Richtung vom Arbeitskolben 68' abstehenden Ringflansches, welcher im Bereich zwischen dem Flansch 54 und der Ringfläche 74' angeordnet oder ausgebildet ist.

Die Regelungseinrichtung 66' stimmt abgesehen von den oben beschriebenen Unterschieden im Aufbau mit der Regelungseinrichtung 66 überein, so dass im Wesentlichen auf die obige Beschreibung in Verbindung mit dem Instrument 10 verwiesen werden kann.

Die Funktionsweise des Instruments 10', insbesondere auch die von der Regelungseinrichtung 66 abweichende Funktionsweise der Regelungseinrichtung 66', wird nachfolgend anhand der Figuren erläutert.

In einer Grundstellung des Instruments 10' ist das Betätigungselement 32 unbetätigt. Die Druckgasquelle 48 steht mit dem Innenraum 178 in Fluidverbindung. Der von der Druckgasquelle 48 bereitgestellte Arbeitsdruck des Druckgases wirkt auf den Ringflansch 124 und drückt diesen gegen die Ringfläche 196. Das Ventil 230 nimmt in der Grundstellung des Instruments 10' eine geschlossene Stellung ein. Das Druckgas kann somit nicht durch die Bohrung 194 hindurchströmen. Das Rückstellglied 76 drückt das Stützelement 80' in proximaler Richtung. Eine Position des Stützelements 80' in der Grundstellung wird vorgegeben durch eine Länge des als Druckfeder ausgebildeten Rückstellglieds 76, welche einen maximalen Abstand zwischen dem Flansch 54 und der Kolbenscheibe 86' definiert. Wie in Figur 8a dargestellt ist das Ende 100' etwas vom Flansch 58 beabstandet. Vorzugsweise ist das Rückstellglied 76 jedoch so lang, dass das Ende 100' am Flansch anliegt. Das Ende 120' nimmt dabei seine proximalste Stellung ein.

Das erste Druckglied 198 drückt die Kolbenscheibe 152 in proximaler Richtung ebenfalls gegen den Ringflansch 124. Das zweite Druckglied 220 drückt das Betätigungsglied 218 in proximaler Richtung gegen die Anschlagfläche 216. Das dritte Druckglied 222 drückt die Ventilscheibe 210 gegen die Dichtelemente 204 und 206 und verschließt die Bohrung 200. Die Bohrung 200 in Verbindung mit der Ventilscheibe 210 und den Dichtelementen 206 und 208 definiert ein Entlüftungsventil 230, welches nur entgegen der Wirkung des dritten Druckglieds 222 geöffnet werden kann. Das Ventil 230 kann nur entgegen der Wirkung des ersten Druckglieds 198 geöffnet werden.

Die vom ersten Druckglied 198 ausgeübte Kraft ist geringfügig größer als die Summe der vom zweiten und dritten Druckglied 220, 222 ausgeübten Kräfte. Wirken dem Arbeitskolben 68' nur geringe Kräfte im Bereich der Werkzeugelemente 234 und 236 definierenden Schneide 26 beziehungsweise des Ambossteils 20 entgegen, so wird in Folge einer Verschwenkung des Betätigungselements 32 in Richtung auf den Griffbereich 30 hin das Betätigungsglied 218 in distaler Richtung bewegt. In diesem ersten oder mechanischen Arbeitsbereich des Instruments 10 erfolgt eine Bewegung des Arbeitskolbens 68' allein aufgrund der mit dem Betätigungselement 32 aufgebrachten Vorschubkraft in distaler Richtung. Die eingeleitete Betätigungskraft wird über das zweite Druckglied 220 und das dritte Druckglied 222 auf die Kolbenscheibe 152 übertragen und mittels der ersten Druckglieds 198 auf die Endfläche 120' des Arbeitskolbens 68', welcher so ohne Unterstützung von Druckgas in distaler Richtung bewegt wird. Dabei wird der Arbeitskolben 68' in distaler Richtung relativ zum Stützelement 80' verschoben, welches durch das Rückstellglied in der Grundstellung gehalten wird. Der erste Arbeitsbereich des Instruments 10' ist in den Figur 8a dargestellt.

Steigt der Widerstand zwischen den Werkzeugelementen 234 und 236 an und übersteigt die dort wirkende Kraft die Summe der vom zweiten Druckglied 220 und vom dritten Druckglied 222 ausgeübten Kräfte, so werden die Druckglieder 220 und 222 komprimiert. Auch das erste Druckglied 198 wird komprimiert, was zur Folge hat, dass das Ventil 230 öffnet und Druckgas vom Innenraum 178 in den zwischen der Endfläche 120' und der Kolbenscheiben 152 definierten Ringraum 238', in dem das erste Druckglied 198 angeordnet ist, einströmen kann. Das Druckgas kann so lange nachströmen bis sich wieder ein Kräftegleichgewicht zwischen der Summe der vom ersten Druckglied 198 ausgeübten Kraft und der durch das Druckgas im Ringraum 238' distalseitig der Kolbenscheibe 152 anliegenden Kraft und der Summe der von den Druckgliedern 220 und 222 an der anderen Seite der Kolbenscheibe 152 ausgeübten Kräfte eingestellt hat. Durch Verschwenken des Betätigungselements 32 auf den Griffbereich 30 hin wird somit sukzessive Druckgas in den Ringraum 238' eingeleitet, welches insbesondere direkt auf die Endfläche 120' des Arbeitskolbens 68' wirkt. Das Ende 100' liegt noch immer am Flansch 58 an.

Der Ringraum 238' steht über die Längsnut 252' mit einem den Zylinderabschnitt 72' ringförmig umgebenden Arbeitsraum 240' in Fluidverbindung. Der Arbeitsraum 240' wird begrenzt durch den Flansch 58, den Arbeitszylinder 52, das Ende 100' und die Ringfläche 244'. Der herrschende Gasdruck wirkt somit auf die in proximaler Richtung weisende Ringfläche 244' des Klemmkolbens 128' sowie auf das Ende 100'.

Ab einem bestimmten Gasdruck im Instrument 10' wird die Feststelleinrichtung 246' aktiviert. Der herrschende Gasdruck wirkt auf das Ende 100' sowie die Ringfläche 244' und bewegt das Stützelement 80' entgegen der Wirkung des Rückstellglieds 76 in distaler Richtung. Der herrschende Gasdruck und die vom Rückstellglied 76 auf das Stützelement 80' ausgeübte Kraft wirken einander entgegen. Sobald die durch das Druckgas im Arbeitsraum 240' auf das Ende 100' wirkende Kraft der vom zunehmend komprimierten Rückstellglied 76 ausgeübten Rückstellkraft entspricht, wirkt der im Arbeitsraum 240' herrschende Gasdruck proximalseitig auf den Klemmkolben 128'. Dieser drückt in distaler Richtung gegen die Klemmelemente 142'. Der Klemmkolben 128' drückt die an ihm anliegende Außenseite der Scheibe 144 in distaler Richtung, wodurch das Stützelement 80' und der Zylinderabschnitt 72' gegeneinander verklemmt werden.

Der Arbeitskolben 68' bildet in der beschriebenen Weise mit dem mittels der Feststelleinrichtung 246' am Zylinderabschnitt 72' festgelegten Stützelement 80' eine Einheit. Der im Instrument 10' herrschende Gasdruck wirkt somit nicht nur wie beschrieben direkt auf den Arbeitskolben 68' über die Endfläche 120', sondern zusätzlich auch noch indirekt über das Stützelement 80', wobei eine im Wesentlichen von der Endfläche 120' definierte Wirkfläche des Arbeitskolben 68' um eine vom Ende 100' sowie der Endfläche 244' definierte, quer zur Längsachse 22 in proximaler Richtung weisende, gestufte Wirkfläche wirkt. Das relativ zum Arbeitszylinder 52 abgedichtete Stützelement 80' bildet somit ab einem bestimmten Gasdruck einen Teil des Arbeitskolbens 68'. Durch die beschriebene Wirkflächenvergrößerung des Arbeitskolbens 68' wird eine auf diesen wirkende Vorschubkraft erhöht, und zwar sobald die Wirkung der Feststelleinrichtung 246' einsetzt.

Bei einer weiteren Bewegung des Betätigungselements 32 in Richtung auf den Griffbereich 30 hin werden die Druckglieder 220 und 222 weiter verkürzt, so dass sich der im Arbeitsraum 240' herrschende Druck bis zu einem Maximum erhöhen kann. Dieser herrschende Druck bewirkt die gewünschte hohe Kraft zwischen den Werkzeugelementen 234 und 236, indem das Druckgas im Ringraum 238' sowie im Arbeitsraum 240' den Arbeitskolben 68' in distaler Richtung drückt.

Wie beschrieben arbeitet das Instrument 10' unterhalb einer vorgegebenen Grenzkraft, die eingestellt werden kann durch entsprechende Auswahl der Druckglieder 198, 220 und 222, im ersten oder mechanischen Arbeitsbereich. Erst oberhalb der Grenzkraft wird durch die Regelungseinrichtung 66' ein zweiter oder pneumatischer Arbeitsbereich definiert, in welchem der Arbeitskolben 68' zusätzlich mit Druckgasunterstützung in distaler Richtung bewegt wird. Dieser zweite Arbeitsbereich ist schematisch in Figur 8b dargestellt. Im zweiten Arbeitsbereich wird das Stützelement 80' dem Arbeitskolben 68' quasi zugeschaltet. Beim Instrument 10 ist das Stützelement 80 zwischengeschaltet und hat eine deutlichere Trennung zwischen mechanischem und druckgasbetriebenem Arbeitsbereich zur Folge.

Auch mit dem Instrument 10' kann so eine gewünschte Maximalkraft im Wesentlichen allein durch das Druckgas aufgebracht werden. Um insbesondere bei der Verwendung eines Druckgasbehälters 42 einen Gasverbrauch auf ein Minimum zu reduzieren, wird das Druckgas durch die Regelungseinrichtung 66' erst bei hohen wirkenden Kräften zugeschaltet.

Der Druckregler 248 stellt sicher, dass ein Arbeitsdruck des Druckgases proportional zu einer auf das Betätigungselement 32 von einer Bedienperson ausgeübten Kraft ist. Damit lässt sich die vom Arbeitskolben 68' ausgeübte Kraft stufenlos erhöhen, wobei dem Anwender so eine größtmögliche Taktilität zur Verfügung steht. Da auch im zweiten oder pneumatischen Arbeitsbereich bedingt durch eine Verkürzung der Druckglieder 220 und 222 ein definierter Weg sowohl des Betätigungselements 32 als auch des Betätigungsglieds 218 zurückgelegt wird, wird auch die Taktilität für den Anwender im zweiten Arbeitsbereich verbessert.

Bei einer Vorwärtsbewegung des Betätigungsglieds 218 ist die Kraft, die das dritte Druckglied 222 ausübt, stets geringfügig größer als die auf die Ventilscheibe 210 wirkende Druckkraft, so dass das Druckgas nicht entweichen kann, insbesondere nicht aus dem Arbeitsraum 240'.

Wird die Kraft auf das Betätigungselement 32 vom Anwender reduziert und dieses vom Griffbereich 30 weg verschwenkt, werden beim Rückhub des Arbeitskolbens 68' die Druckglieder 220 und 222 verlängert, so dass die vom dritten Druckglied 222 ausgeübte Kraft nicht mehr ausreicht, um die Bohrung 200 zu verschließen. Der Druck im Instrument 10' fällt ab, indem das Druckgas über das Entlüftungsventil 232 entweicht, bis die Kraft des dritten Druckglieds 222 wieder ausreicht, um die Bohrung 200 zu verschließen. Auf diese Weise fällt der Druck im Arbeitsraum 240' proportional zum zurückgelegten Weg des Betätigungsglieds 218 in proximaler Richtung ab, wenn auch mit einer gewissen Rücksteuerhysterese.

Um das Instrument 10' vor Überdruck zu schützen, kann zusätzlich ein in den Figuren nicht dargestelltes Überdruckventil vorgesehen sein, welches in Fluidverbindung mit dem Innenraum 178 steht.

Bei den beschriebenen Instrumenten 10 und 10' kann über den größten Teil des vom Arbeitskolben 68 beziehungsweise 68' zurückgelegten Weges, auf dem keine oder nur eine kleine Kraft zwischen den Werkzeugelementen 234 und 236 wirkt, die Kraft mechanisch vom Anwender aufgebracht werden. Erst auf dem letzten Stück des Weges wird die volle Arbeitskraft durch Druckgasbeaufschlagung aufgebracht. Bei üblichen Maulweiten von Knochenstanzen von 20 mm und üblichen Arbeitswegen von circa 5 mm, also nur etwa ein Viertel des möglichen Gesamtweges oder -hubes des Arbeitskolbens 68, wird nur auf einem Viertel des Gesamthubs der Arbeitskolben 68 beim erfindungsgemäßen Instrument pneumatisch verschoben, auf drei Viertel des Weges rein mechanisch. Dadurch lässt sich der Gasverbrauch auf etwa ein Viertel reduzieren. Mit der gleichen Gasmenge können somit etwa viermal so viele Betätigungen der erfindungsgemäßen Instrumente 10 und 10' mit maximaler Arbeitskraft erfolgen als bei Instrumenten ohne die Regelungseinrichtungen 66 beziehungsweise 66'.

Der variabel einstellbare Druckregler 248 ermöglicht also sowohl beim Instrument 10 als auch beim Instrument 10' eine stufenlose Veränderung des Arbeitsdrucks während der Bedienung. Dies gewährleistet ein Maximum an Taktilität. Ferner wird durch die beschriebene Kombination von mechanischem und pneumatischem Vorschub der Gasverbrauch auf ein Minimum reduziert. Dadurch können im Vergleich zu herkömmlichen Instrumenten deutlich kleinere Druckgasbehälter 42 verwendet werden, wodurch sich das vom Gehäuse 38 definierte Volumen im Vergleich zu bekannten Instrumenten deutlich verkleinern lässt.

## Patentansprüche

1. Druckgasbetriebenes chirurgisches Instrument (10; 10') mit einem Druckgasanschluss (44) zum Verbinden mit einer Druckgasquelle (48), einem mit einem Druckgas beaufschlagbaren Arbeitskolben (68; 68'), einem Betätigungselement (32) zum Betätigen des Instruments (10; 10') und einer mit dem Betätigungselement (32) und dem Arbeitskolben (68; 68') gekoppelten Regelungseinrichtung (66; 66') zum Regeln einer Vorschubkraft des Arbeitskolbens (68; 68'), **dadurch gekennzeichnet, dass** die Regelungseinrichtung (66; 66') derart ausgebildet ist, dass sie einen ersten Arbeitsbereich, in welchem der Arbeitskolben (68; 68') allein aufgrund einer mit dem Betätigungselement (32) aufbringbaren Vorschubkraft in distaler Richtung bewegbar ist, für Vorschubkräfte unterhalb einer vorgegebenen Grenzkraft definiert, und dass die Regelungseinrichtung (66; 66') einen zweiten Arbeitsbereich oberhalb der Grenzkraft definiert, in welchem der Arbeitskolben (68; 68') durch Beaufschlagen mit Druckgas in distaler Richtung bewegbar ist.

2. Chirurgisches Instrument nach Anspruch 1, **dadurch gekennzeichnet, dass** die Regelungseinrichtung (66; 66') derart ausgebildet ist, dass im zweiten Arbeitsbereich eine durch Beaufschlagen mit Druckgas auf den Arbeitskolben (68; 68') wirkende Vorschubkraft proportional zu einer mit dem Betätigungselement (32) aufbringbaren Vorschubkraft ist.

3. Chirurgisches Instrument nach einem der voranstehenden Ansprüche, gekennzeichnet durch ein Stützelement (80; 80'), welches mit dem Arbeitskolben (68; 68') im ersten und/oder zweiten Arbeitsbereich zusammenwirkt.

4. Chirurgisches Instrument nach Anspruch 3, **dadurch gekennzeichnet, dass** sich der Arbeitskolben (68) im ersten Arbeitsbereich am Stützelement abstützt.

5. Chirurgisches Instrument nach Anspruch 3 oder 4, **dadurch gekennzeichnet, dass** der Arbeitskolben (68') im zweiten Arbeitsbereich mit dem Stützelement (80') gekoppelt ist.

6. Chirurgisches Instrument nach einem der Ansprüche 3 bis 5, **dadurch gekennzeichnet, dass** das Stützelement (80) im ersten Arbeitsbereich nur aufgrund der mit dem Betätigungselement (32) aufbringbaren Vorschubkraft in distaler Richtung bewegbar ist.

7. Chirurgisches Instrument nach einem der Ansprüche 3 bis 6, **dadurch gekennzeichnet, dass** die Regelungseinrichtung (66; 66') eine Feststelleinrichtung (246; 246') zum temporären Festlegen des Stützelements (80; 80') im zweiten Arbeitsbereich umfasst.

8. Chirurgisches Instrument nach Anspruch 7, **dadurch gekennzeichnet, dass** die Feststelleinrichtung (246; 246') mindestens ein Feststellglied (142; 142') umfasst zum temporären Festlegen des Stützelements (80; 80') an der Führungseinrichtung (250) oder dem Arbeitskolben (68').

9. Chirurgisches Instrument nach einem der voranstehenden Ansprüche, **dadurch gekennzeichnet, dass** die Regelungseinrichtung (66; 66') einen Druckregler (248) umfasst zum Regeln eines auf den Arbeitskolben (68; 68') wirkenden Gasdrucks in Abhängigkeit einer über das Betätigungselement (32) einleitbaren Betätigungskraft.

10. Chirurgisches Instrument nach einem der voranstehenden Ansprüche, **dadurch gekennzeichnet, dass** die über das Betätigungselement (32) eingeleitete Betätigungskraft auf ein parallel zum Arbeitskolben (68; 68') bewegbar gelagertes Betätigungsglied (218) übertragbar ist und dass die Regelungseinrichtung (66; 66') ein zweites Druckglied (220) umfasst zum Übertragen einer Betätigungskraft vom Betätigungsglied (218) auf den Druckregler (248).

11. Chirurgisches Instrument nach einem der voranstehenden Ansprüche, **dadurch gekennzeichnet, dass** die über das Betätigungselement (32) eingeleitete Betätigungskraft auf ein parallel zum Arbeitskolben (68; 68') bewegbar gelagertes Betätigungsglied (218) übertragbar ist und dass die Regelungseinrichtung (66; 66') ein drittes Druckglied (222) umfasst, zum Übertragen einer Betätigungskraft vom Betätigungsglied (218) auf den Druckregler (248).

12. Chirurgisches Instrument nach einem der voranstehenden Ansprüche, **dadurch gekennzeichnet, dass** die Regelungseinrichtung (66) einen mit Druckgas beaufschlagbaren Arbeitsraum (240) umfasst, welcher in Richtung auf den Arbeitskolben (68) hin mindestens teilweise offen ist, und dass die Regelungseinrichtung (66; 66') ein Belüftungsventil (232) umfasst zum Belüften des Arbeitsraumes (240).

13. Chirurgisches Instrument nach einem der voranstehenden Ansprüche, gekennzeichnet durch eine Rückstelleinrichtung (78) zum Überführen des Instruments (10; 10') von einer Arbeitsstellung, in welcher der Arbeitskolben (68; 68') in distaler Richtung bewegt ist, in eine Grundstellung zurück, in welcher der Arbeitskolben (68; 68) seine proximalste Stellung einnimmt.

14. Chirurgisches Instrument nach einem der voranstehenden Ansprüche, **dadurch gekennzeichnet, dass** die Druckgasquelle (48) ein mit einem Druckgas gefüllter Gasbehälter (42) ist und dass der Gasbehälter (42) mit dem Druckgasanschluss (44) lösbar verbindbar ist.

15. Chirurgisches Instrument nach einem der voranstehenden Ansprüche, **dadurch gekennzeichnet, dass** das Instrument (10; 10') in Form einer Knochenstanze oder in Form eines Implantatverriegelungsinstruments ausgebildet ist.

## Claims

1. Surgical instrument (10; 10') operated by pressurized gas, having a pressurized gas connection (44) for connection to a source of pressurized gas (48), a working piston (68; 68') adapted to be acted upon by a pressurized gas, an actuating element (32) for actuating the instrument (10; 10'), and a regulating device (66; 66') coupled to the actuating element (32) and to the working piston (68; 68') for regulating a feed force of the working piston (68; 68'), **characterized in that** the regulating device (66; 66') is configured such that for feed forces below a predetermined limit force it defines a first work area in which the working piston (68; 68') is movable in the distal direction solely owing to a feed force that can be applied with the actuating element (32), and **in that** above the limit force the regulating device (66; 66') defines a second work area in which the working piston (68; 68') is movable in the distal direction by being acted upon by pressurized gas.

2. Surgical instrument in accordance with Claim 1, **characterized in that** the regulating device (66; 66') is configured such that in the second work area a feed force acting on the working piston (68; 68') as a result of it being acted upon by pressurized gas is proportional to a feed force that can be applied with the actuating element (32).

3. Surgical instrument in accordance with any one of the preceding claims, **characterized by** a supporting element (80; 80') which interacts with the working piston (68; 68') in the first and/or second work area.

4. Surgical instrument in accordance with Claim 3, **characterized in that** the working piston (68) is supported in the first work area on the supporting element.

5. Surgical instrument in accordance with Claim 3 or 4, **characterized in that** the working piston (68') is coupled in the second work area to the supporting element (80').

6. Surgical instrument in accordance with any one of Claims 3 to 5, **characterized in that** the supporting element (80) is movable in the distal direction in the first work area solely owing to the feed force that can be applied with the actuating element (32).

7. Surgical instrument in accordance with any one of Claims 3 to 6, **characterized in that** the regulating device (66; 66') comprises a locking device (246; 246') for temporarily securing the supporting element (80; 80') in the second work area.

8. Surgical instrument in accordance with Claim 7, **characterized in that** the locking device (246; 246') comprises at least one locking member (142; 142') for temporarily securing the supporting element (80; 80') to the guiding device (250) or to the working piston (68').

9. Surgical instrument in accordance with any one of the preceding Claims, **characterized in that** the regulating device (66; 66') comprises a pressure regulator (248) for regulating a gas pressure acting on the working piston (68; 68') in dependence upon an actuating force introducible via the actuating element (32).

10. Surgical instrument in accordance with any one of the preceding Claims, **characterized in that** the actuating force introduced via the actuating element (32) is transmittable to an actuating member (218) mounted so as to be movable parallel to the working piston (68; 68') and **in that** the regulating device (66; 66') comprises a second pressure member (220) for transmitting an actuating force from the actuating member (218) to the pressure regulator (248).

11. Surgical instrument in accordance with any one of the preceding Claims, **characterized in that** the actuating force introduced via the actuating element (32) is transmittable to an actuating member (218) mounted so as to be movable parallel to the working piston (68; 68') and **in that** the regulating device (66; 66') comprises a third pressure member (222) for transmitting an actuating force from the actuating member (218) to the pressure regulator (248).

12. Surgical instrument in accordance with any one of the preceding Claims, **characterized in that** the regulating device (66) comprises a work space (240) adapted to be acted upon by pressurized gas, which is at least partially open in the direction towards the working piston (68) and **in that** the regulating device (66; 66') comprises a ventilation valve (232) for ventilating the work space (240).

13. Surgical instrument in accordance with any one of the preceding claims, **characterized by** a resetting device (78) for transferring the instrument (10; 10') from a working position into which the working piston (68; 68') has been moved in the distal direction back into a basic position in which the working piston (68; 68') assumes its most proximal position.

14. Surgical instrument in accordance with any one of the preceding claims, **characterized in that** the source of pressurized gas (48) is a gas container (42) filled with a pressurized gas, and **in that** the gas container (42) is releasably connectable to the pressurized gas connection (44).

15. Surgical instrument in accordance with any one of the preceding claims, **characterized in that** the instrument (10; 10') is configured in the form of a bone punch or in the form of an implant locking instrument.

## Revendications

1. Instrument médical (10; 10') fonctionnant au gaz sous pression, qui comporte,
un raccord de gaz sous pression (44) destiné à être relié à une source de gaz sous pression (48),
un piston de travail (68; 68') pouvant être alimenté et sollicité par un gaz sous pression,
un élément d'actionnement (32) pour actionner l'instrument (10; 10'), et
un dispositif de régulation (66; 66') couplé à l'élément d'actionnement (32) et au piston de travail (68; 68') en vue de réguler une force d'avance du piston de travail (68; 68'),
**caractérisé en ce que** le dispositif de régulation (66; 66') est conçu de manière à définir une première plage de travail, dans laquelle le piston de travail (68; 68') peut être déplacé en direction distale uniquement sous l'effet d'une force d'avance pouvant être appliquée par l'élément d'actionnement (32), pour des forces d'avance en-dessous d'une force limite prescrite, et **en ce que** le dispositif de régulation (66; 66') définit une deuxième plage de travail au-dessus de ladite force limite, dans laquelle le piston de travail (68; 68') peut être déplacé en direction distale en étant alimenté et sollicité par du gaz sous pression.

2. Instrument chirurgical selon la revendication 1, **caractérisé en ce que** le dispositif de régulation (66; 66') est conçu de manière à ce que dans la deuxième plage de travail, une force d'avance agissant sur le piston de travail (68; 68') par l'alimentation en gaz sous pression, est proportionnelle à une force d'avance pouvant être appliquée avec l'élément d'actionnement (32).

3. Instrument chirurgical selon l'une des revendications précédentes, **caractérisé par** un élément d'appui (80; 80'), qui interagit avec le piston de travail (68; 68') dans la première et/ou la deuxième plage de travail.

4. Instrument chirurgical selon la revendication 3, **caractérisé en ce que** le piston de travail (68) s'appuie contre l'élément d'appui, dans la première plage de travail.

5. Instrument chirurgical selon la revendication 3 ou la revendication 4, **caractérisé en ce que** le piston de travail (68') est couplé à l'élément d'appui (80'), dans la deuxième plage de travail.

6. Instrument chirurgical selon l'une des revendications 3 à 5, **caractérisé en ce que** dans la première plage de travail, l'élément d'appui (80) ne peut être déplacé en direction distale que sous l'effet de la force d'avance pouvant être appliquée à l'aide de l'élément d'actionnement (32).

7. Instrument chirurgical selon l'une des revendications 3 à 6, **caractérisé en ce que** le dispositif de régulation (66; 66') comprend un dispositif d'immobilisation (246; 246') pour l'immobilisation temporaire de l'élément d'appui (80; 80') dans la deuxième plage de travail.

8. Instrument chirurgical selon la revendication 7, **caractérisé en ce que** le dispositif d'immobilisation (246; 246') comporte au moins un organe d'immobilisation (142; 142') pour l'immobilisation temporaire de l'élément d'appui (80; 80') sur le dispositif de guidage (250) ou le piston de travail (68').

9. Instrument chirurgical selon l'une des revendications précédentes, **caractérisé en ce que** le dispositif de régulation (66; 66') comprend un régulateur de pression (248) pour réguler une pression de gaz agissant sur le piston de travail (68; 68'), en fonction d'une force d'actionnement pouvant être provoquée par l'intermédiaire de l'élément d'actionnement (32).

10. Instrument chirurgical selon l'une des revendications précédentes, **caractérisé en ce que** la force d'actionnement provoquée par l'intermédiaire de l'élément d'actionnement (32) peut être transmise à un organe d'actionnement (218) monté de manière à pouvoir se déplacer en parallèle au piston de travail (68; 68'), et **en ce que** le dispositif de régulation (66; 66') comprend un deuxième organe de compression (220) pour transmettre une force d'actionnement de l'organe d'actionnement (218) au régulateur de pression (248).

11. Instrument chirurgical selon l'une des revendications précédentes, **caractérisé en ce que** la force d'actionnement provoquée par l'intermédiaire de l'élément d'actionnement (32) peut être transmise à un organe d'actionnement (218) monté de manière à pouvoir se déplacer en parallèle au piston de travail (68; 68'), et **en ce que** le dispositif de régulation (66; 66') comprend un troisième organe de compression (222) pour transmettre une force d'actionnement de l'organe d'actionnement (218) au régulateur de pression (248).

12. Instrument chirurgical selon l'une des revendications précédentes, **caractérisé en ce que** le dispositif de régulation (66) comprend une chambre de travail (240) pouvant être alimentée en gaz sous pression et au moins partiellement ouverte en direction du piston de travail (68), et **en ce que** le dispositif de régulation (66; 66') comporte une valve de ventilation (232) pour ventiler la chambre de travail (240).

13. Instrument chirurgical selon l'une des revendications précédentes, **caractérisé par** un dispositif de rappel (78) pour transférer l'instrument (10; 10') d'une position de travail, dans laquelle le piston de travail (68; 68') est déplacé dans la direction distale, en retour à une position de base dans laquelle le piston de travail (68; 68) prend sa position proximale.

14. Instrument chirurgical selon l'une des revendications précédentes, **caractérisé en ce que** la source de gaz sous pression (48) est un récipient de gaz (42) rempli avec un gaz sous pression, et **en ce que** le récipient de gaz (42) peut être relié de manière amovible au raccord de gaz sous pression (44).

15. Instrument chirurgical selon l'une des revendications précédentes, **caractérisé en ce que** l'instrument (10; 10') est réalisé sous la forme d'une poinçonneuse d'os chirurgicale ou sous la forme d'un instrument de verrouillage d'implant.
